# EUROPEAN PATENT SPECIFICATION

(11) **EP 4 103 170 B1**
(45) Date of publication and mention of the grant of the patent: **08.04.2026**
(21) Application number: 21704272.0
(22) Date of filing: 10.02.2021
(51) Int. Cl.: A61K 31/201, A61K 31/202, A61K 38/17, A61K 45/06, A61P 35/00

(54) **A PHARMACEUTICAL COMBINATION FOR THE TREATMENT OF A CANCER**
PHARMAZEUTISCHE KOMBINATIONEN ZUR BEHANDLUNG VON KREBS
COMBINAISON PHARMACEUTIQUE POUR LE TRAITEMENT DU CANCER

(30) Priority: 10.02.2020 EP 20382089
(43) Date of publication of application: 21.12.2022
(73) Proprietor: Ability Pharmaceuticals S.A., 08193 Cerdanyola del Vallès (ES)
(72) Inventor: PÉREZ MONTOYO, Héctor, 08193 CERDANYOLA DEL VALLÈS (ES); YESTE-VELASCO, Marc, 08193 CERDANYOLA DEL VALLÈS (ES); MUÑOZ GUARDIOLA, Pau, 08193 CERDANYOLA DEL VALLÈS (ES); ALFÓN CORIAT, José Alberto, 08193 CERDANYOLA DEL VALLÈS (ES); DOMÈNECH GARCIA, Carles, 08193 CERDANYOLA DEL VALLÈS (ES); YOLDI SALINAS, Guillermo, 08193 CERDANYOLA DEL VALLÈS (ES); LIZCANO DE LA VEGA, Jose Miguel, 08193 CERDANYOLA DEL VALLÈS (ES); SEGURA GUINARD, Miguel Francisco, 08193 CERDANYOLA DEL VALLÈS (ES); PARIS-CODERCH, Laia, 08193 CERDANYOLA DEL VALLÈS (ES); FESTUCCIA, Claudio, 08193 CERDANYOLA DEL VALLÈS (ES)
(74) Representative: ZBM Patents - Zea, Barlocci & Markvardsen
(86) International application number: PCT/EP2021/053162
(87) International publication number: WO 2021/160650

(56) References cited:
- EP-B1- 2 409 963
- WO-A1-2013/050644
- WO-A1-2018/210830
- DANIEL H. LOPEZ ET AL: "2-Hydroxy Arachidonic Acid: A New Non-Steroidal Anti-Inflammatory Drug", PLOS ONE, vol. 8, no. 8, 27 August 2013 (2013-08-27), pages e72052, XP055345829, DOI: 10.1371/journal.pone.0072052

## Description

### FIELD OF THE INVENTION

The present invention relates to use of ABTL0812 in the treatment of a cancer in a human patient, wherein the cancer treatment is related to chemotherapy, targeted therapy treatment, immunotherapy treatment or radiotherapy treatment.

### BACKGROUND ART

EP2409963B1 (Lipopharma - filed in 2010) describes use of 1,2-derivatives of polyunsaturated fatty acids (termed D-PUFAs) compounds for treatment of cancer.

The described fatty acids derivative compounds have the following formula:

COO***R₁***-CH***R₂***-(CH2)***a***-(CH=CH-CH₂)*b*-(CH₂)***c***-CH₃

An example of a preferred compound is:

COOH-CHOH-(CH₂)₆-(CH=CH-CH₂)₂-(CH₂)₃-CH₃ (182A1)

The article "Erazo, et al.; Clinical Cancer Research; 22(10) May 15, 2016" describes the above referred compound (182A1) in further details - in the article is this compound termed "ABTL0812" and this term is used herein.

As known in the art - the pharmacological treatment of cancer is generally based in four main groups of drugs including chemotherapy, targeted therapy, hormone therapy and immunotherapy. In addition, radiotherapy is also at a cornerstone of cancer treatment that is many times administered together with pharmacotherapy.

WO2018/210830A1 (Ability Pharmaceuticals) describes use of the ABTL0812 compound in combination with other chemotherapeutic agents in the treatment of a cancer - such as e.g. a pharmaceutical combination of ABTL0812 with the chemotherapeutic agents Docetaxel, Paclitaxel, Carboplatin or Cisplatin in relation to first-line therapy.

### SUMMARY OF THE INVENTION

The invention is set out in the appended set of claims.

Any subject-matter falling outside the scope of the claims is provided for information purposes only.

Starting with WO2018/210830A1 (Ability Pharmaceuticals) as most relevant prior art document (so-called closest prior art document) - the problem to be solved by the present invention may be seen as the provision of alternative uses of ABTL0812 that may give an improved treatment of cancer.

As discussed above, the compound COOH-CHOH-(CH₂)₆-(CH=CH-CH₂)₂-(CH₂)₃-CH₃ is herein termed ABTL0812.

As discussed above, WO2018/210830A1 (Ability Pharmaceuticals) describes use of the ABTL0812 compound in combination with other chemotherapeutic agents in the treatment of a cancer - such as e.g. a pharmaceutical combination of ABTL0812 with the chemotherapeutic agents Docetaxel, Paclitaxel, Carboplatin or Cisplatin in relation to first-line therapy.

WO2018/210830A1 (Ability Pharmaceuticals) does not directly and unambiguously describe use of ABTL0812 in second-line therapy for treatment of cancer - for instance are the terms "second-line" or "second line" in relation to second-line therapy not even mentioned in WO2018/210830A1.

Working examples herein provides detailed experimental data plausible demonstrating a significant synergistic effect in relation to use of the above discussed ABTL0812 compound in combination with other chemotherapeutic agents for e.g. second-line therapy treatment of a cancer in a human patient.

As known in the art, first-line therapy is the treatment regimen or regimens that are generally accepted by the medical establishment for initial treatment of a given type and stage of cancer. It is also called primary treatment or therapy. The intent of first-line therapy is to cure the cancer if possible. Also called induction therapy, this primary therapy is the first assault of chemotherapy drugs on the malignancy.

In agreement with the common general knowledge of the skilled person - the term "second-line therapy" of the first aspect relates to second-line therapy treatment tried when the first-line therapy does not work adequately. The management of a cancer case requires regular evaluation of treatment and adjustment as needed. A break with the primary first-line therapy treatment and an adoption of a new regimen signals "second-line therapy" treatment.

As understood by the skilled person in the present context - the term "second-line therapy" requires that the patient in the first-line therapy has been treated with a profile/mixture of cancer agent(s) that is/are different from the profile/mixture of cancer agent(s) of the "second-line therapy". One may say that if the profile/mixture of cancer agent(s) of the first-line therapy had worked satisfactory (i.e. cured the cancer of the patient), then would it probably not be required to use a "second-line therapy".

Just as an example - in the present context could a first-line therapy e.g. have involved use of Docetaxel, Paclitaxel and maybe also ABTL0812 and a second-line therapy could then be a different profile/mixture of cancer agent(s) that e.g. could be ABTL0812 in combination with Temozolomide as discussed in e.g. working Example 1.1 herein.

According to the art, chemotherapy is a type of cancer treatment that uses one or more anti-cancer drugs (chemotherapeutic agents) as part of a standardized chemotherapy regimen. The term chemotherapy has come to connote non-specific usage of intracellular toxic compound to inhibit mitosis, cell division - i.e. a chemotherapeutic agent compound is understood to be a compound that interfere with cell replication. Because DNA/cell replication is a common process that all cells use when they want to make more copies of themselves, chemotherapies cannot distinguish between cancerous and normal cells. Thus, classic chemotherapy may have significant side effects.

As discussed above, WO2018/210830A1 (Ability Pharmaceuticals) describes use of the ABTL0812 compound in combination with other chemotherapeutic agents in the treatment of a cancer - accordingly, this document does not directly and unambiguously describe use of ABTL0812 in targeted therapy, immunotherapy and/or radiotherapy for treatment of cancer.

Working examples herein provides detailed experimental data plausible demonstrating a significant positive effect in relation to use of the above discussed ABTL0812 compound in targeted therapy, immunotherapy or radiotherapy for treatment of cancer in a human patient.

As known in the art, targeted therapy or molecularly targeted therapy is one of the major modalities of medical treatment (pharmacotherapy) for cancer, others being e.g. cytotoxic chemotherapy. As a form of molecular medicine, targeted therapy blocks the growth of cancer cells by interfering with specific targeted molecules needed for carcinogenesis and tumor growth, rather than by simply interfering with all dividing cells (e.g. with traditional chemotherapy).

An aspect of the invention relates to a pharmaceutical combination comprising:
((A): a compound which is a polyunsaturated fatty acid of formula COO**R₁**-CH**R₂**-(CH₂)**a-**(CH=CHCH₂)**b**-(CH₂)**c**-CH₃, a pharmaceutically acceptable salt thereof, or a combination thereof, wherein
   (i) **a** can be any integer value between 0 and 7,
   (ii) **b** can be any integer value between 2 and 7,
   (iii) **c** can be any integer value between 0 to 7,
   (iv) **R₁** is H, Na, K, CH₃, CH₃-CH₂, or PO(O-CH₂-CH₃)₂, and
   (v) **R₂** is OH, OCH₃, O-CH₂COOH, CH₃, Cl, CH₂OH, OPO(O-CH₂-CH₃)₂, N(OH)₂, F, HCOO or N(OCH₂CH₃)₂;
   and
(B3): an immunotherapy agent compound
   for the simultaneous, separate or sequential use in the treatment of a cancer in a human patient, wherein the treatment is an immunotherapy treatment of a cancer,
   wherein (B3) is an immunotherapy agent compound which is a checkpoint inhibitor selected from the group consisting of:
      - an anti-PD1 antibody, particularly wherein the anti-PD1 antibody is Nivolumab, Pembrolizumab or Spartalizumab; and
      - an anti-PDL1 antibody, particularly wherein the anti-PDL1 antibody is Atezolizumab, Avelumab or Durvalumab; and
   wherein Compound (A) is COOH-CHOH-(CH₂)₆-(CH=CH-CH₂)₂-(CH₂)₃-CH₃ (ABTL0812) or a pharmaceutically acceptable salt thereof.

The term "immunotherapy" should be understood according to the art.

As known in the art, immunotherapy is the treatment of disease by activating or suppressing the immune system. Immunotherapies designed to elicit or amplify an immune response are classified as activation immunotherapies, while immunotherapies that reduce or suppress are classified as suppression immunotherapies. In recent years, immunotherapy has become of great interest to researchers, clinicians and pharmaceutical companies, particularly in its promise to treat various forms of cancer.

As known in the art, radiotherapy (also called radiation therapy) is a cancer treatment that uses high doses of radiation to kill cancer cells and shrink tumors.

When there herein is generically referred to Compound (B) it is understood to refer to any of Compound (B1), Compound (B2) and/or Compound (B3).

As understood by the skilled person in the present context - the agent of Compound (B) of the relevant aspects above is of course not a compound within the scope of Compound (A) of the relevant aspects above.

As understood by the skilled person in the present context - in relation to the herein discussed combination treatment it is not essential if the two Compounds (A) and (B) are administered e.g. simultaneous as a single composition or e.g. sequentially as two separate compositions. The important matter is that an effective amount of the compound/agent first administered is in the patient's body and/or has exerted its effect in the patient's body when the second compound/agent is administered.

As understood by the skilled person in the present context - the aspects of the present invention relate to the combination of Compound (A) and at least one Compound (B) e.g. the combination of Compound (A)+Compound (B1), Compound (A)+Compound (B2), Compound (A)+Compound (B3), or Compound (A)+Compound (B1)+Compound (B3). It is also understood that since Compound (A) can be administered in combination with radiotherapy according to the fourth aspect of the invention, radiotherapy can also be administered in combination with the above mentioned combinations with any Compound (B).

Accordingly, the term "combination" of the relevant aspects above relates herein to the various combinations of Compounds (A) and (B), for example in a single pharmaceutical composition, in a combined mixture composed from separate pharmaceutical formulations/compositions of the single active compounds, such as a "tank-mix", and in a combined use of the single active ingredients when applied in a sequential manner, i.e. one after the other with a reasonably short period, such as a few hours or days or in simultaneous administration. The order of applying the Compounds (A) and (B) is not essential.

A combination of the Compounds (A) and (B) can be formulated for its simultaneous, separate or sequential administration. Particularly, if the administration is not simultaneous, the compounds are administered in a relatively close time proximity to each other. Furthermore, compounds are administered in the same or different dosage form or by the same or different administration route, e.g. one compound can be administered intravenously and the other compound can be administered orally. The combination of the two compounds can e.g. be administered:
- as a combination that is being part of the same medicament formulation, the two compounds being then administered always simultaneously;
- as a combination of two units/compositions, each with one of the substances giving rise to the possibility of simultaneous, sequential or separate administration;

For instance, the Compound (A) is independently administered from the compound (B) (i.e. in two units) but at the same time.

In another suitable example, the Compound (A) is administered first and then the compound (B) is separately or sequentially administered - alternatively, the Compound (B) is administered first and then the Compound (A) is separately or sequentially administered.

In another suitable example when two Compounds (B) are administered, the Compound (A) is administered first, a first Compound (B) is separately or sequentially administered secondly and then a second Compound (B) is separately or sequentially administered thirdly. Alternatively, a first Compound (B) is administered first, a second Compound (B) is separately or sequentially administered secondly and then the Compound (A) is separately or sequentially administered thirdly. Alternatively, a first compound (B) is administered first, the Compound (A) is separately or sequentially administered secondly and then a second Compound (B) is separately or sequentially administered thirdly.

The term "pharmaceutical" e.g. in relation to a "pharmaceutical composition" shall be understood according to the art - i.e. that it refers to a preparation/composition which is in such form as to permit the biological activity of the active ingredients to be effective, and physiologically tolerable, that is, which contains no additional components which are unacceptably toxic to a subject to which the composition would be administered. Particularly, the term "pharmaceutically acceptable" means it is approved by a regulatory agency of a state or federal government or is included in the U.S. Pharmacopoeia or other generally recognized pharmacopoeia for use in animals, and more particularly in humans.

Embodiment of the present invention is described below, by way of examples only.

A combination of a herein described preferred embodiment with another herein described preferred embodiment is an even more preferred embodiment.

### DRAWINGS

### ABTL0812 and ABTL are used indistinguishably in this description.

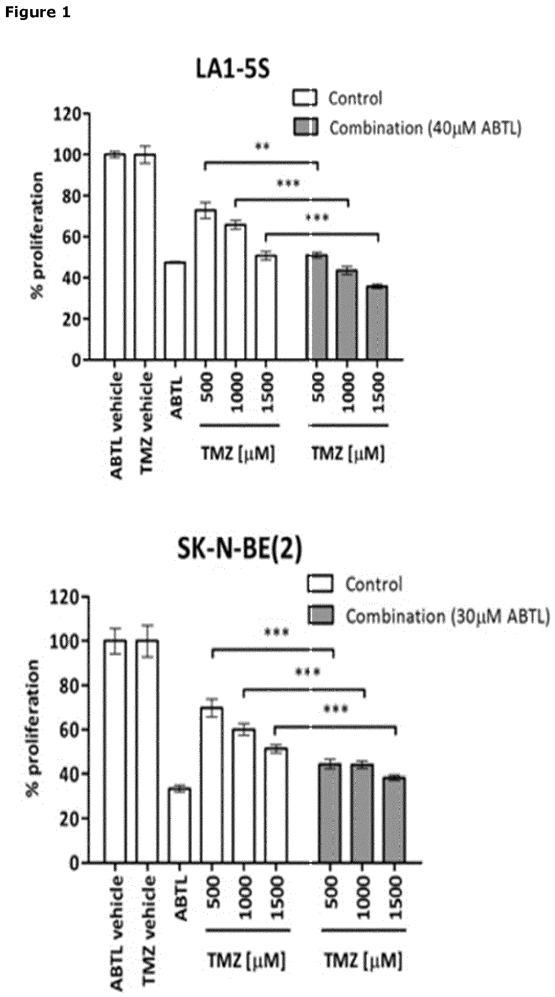

Figure 1: Cytotoxicity of ABTL0812 (ABTL) and temozolomide (TMZ) in LA1-5S and SK-N-BE(2) cells. See working Example herein for further details.

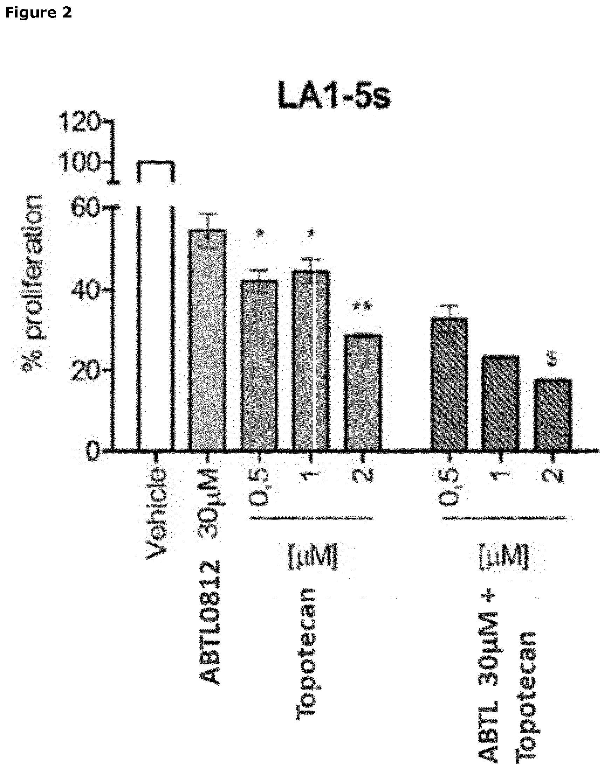

Figure 2: Cytotoxicity of ABTL0812 and topotecan in LA1-5S. See working Example herein for further details.

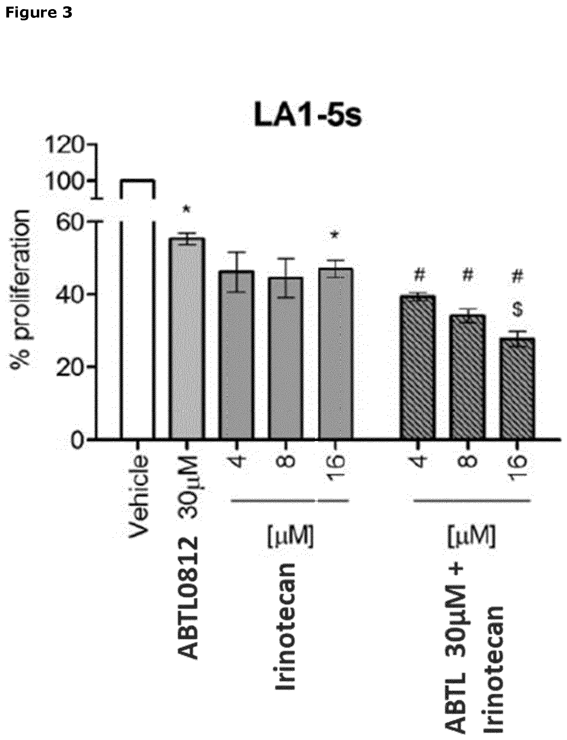

Figure 3: Cytotoxicity of ABTL0812 and irinotecan in LA1-5S. See working Example herein for further details.

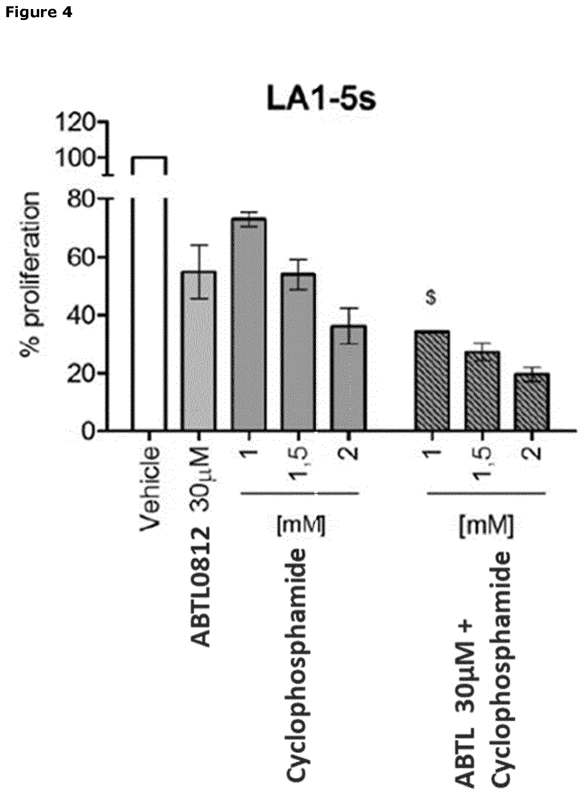

Figure 4: Cytotoxicity of ABTL0812 and cyclophosphamide in LA1-5S. See working Example herein for further details.

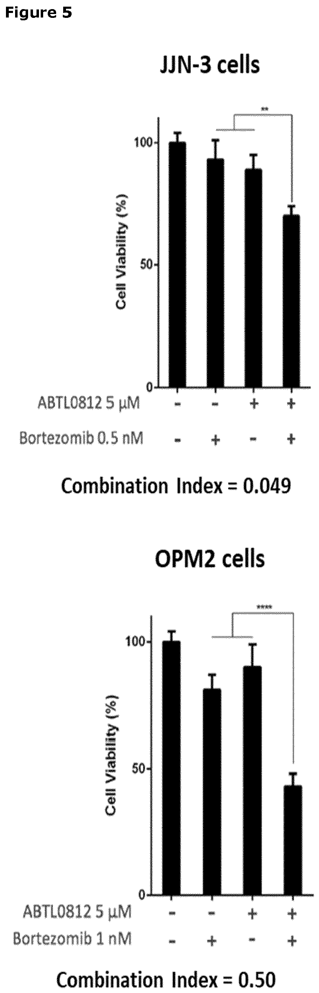

Figure 5: ABTL0812 and bortezomib have strong synergistic effects *in vitro* in the multiple myeloma cell lines JJN-3 and OPM2. See working Example herein for further details.

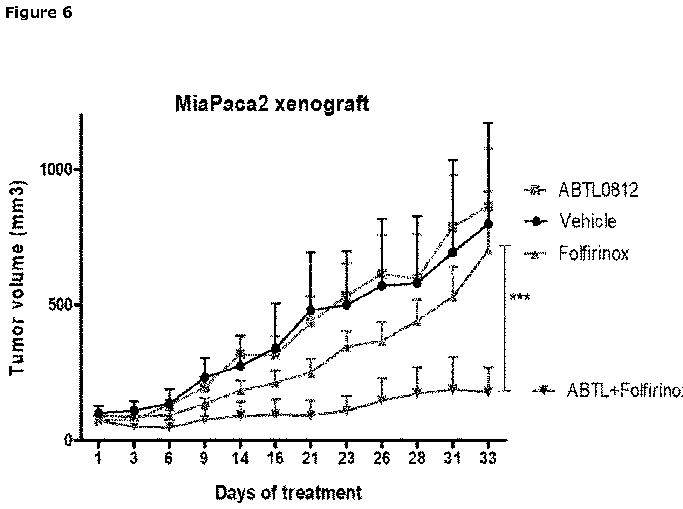

Figure 6: ABTL0812 significantly potentiates Folfirinox anticancer effects without increasing toxicity in a human pancreatic cancer xenograft model using MiaPaca2 cells implanted in nude mice. Results suggests that the combinatory treatment could have a clinical interest for the treatment of pancreatic cancer. See working Example herein for further details.

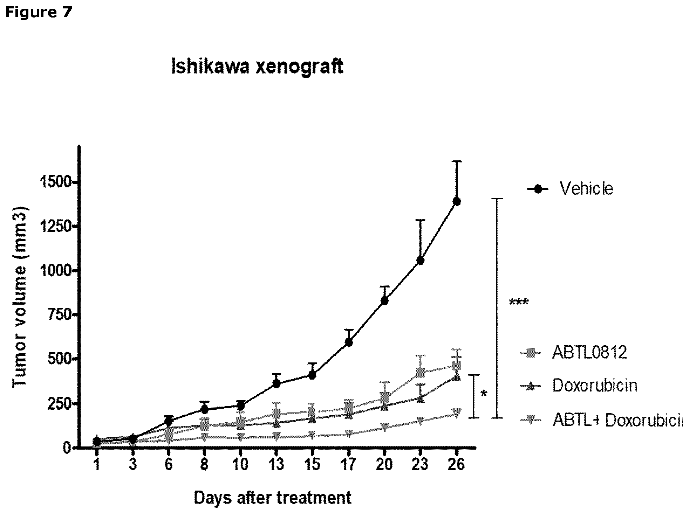

Figure 7: ABTL0812 significantly potentiates doxorubicin anticancer effects without increasing toxicity in a human endometrial cancer xenograft model using Ishikawa cells implanted in nude mice Results suggest a combined therapy of ABTL0812 plus Doxorubicin could have a clinical interest for the treatment of endometrial cancer. See working Example herein for further details.

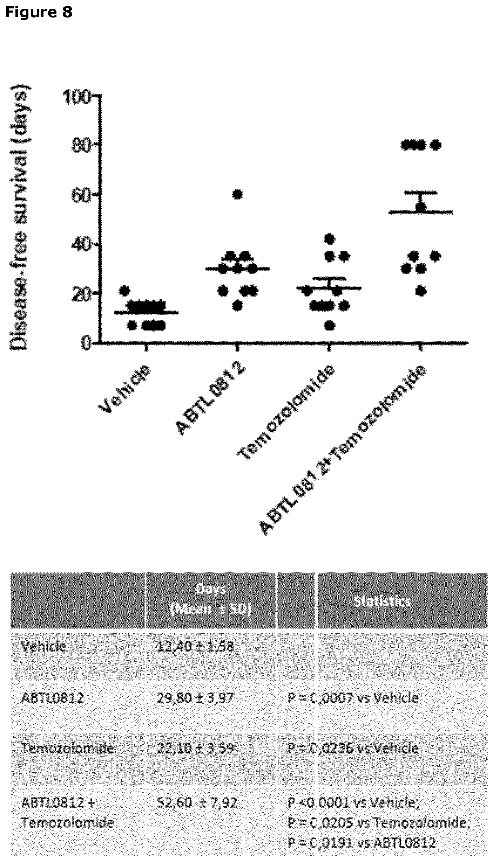

Figure 8: ABTL0812 increases the disease-free survival of mice bearing glioblastoma tumors and potentiates the antitumor activity of temozolomide. See working Example herein for further details.

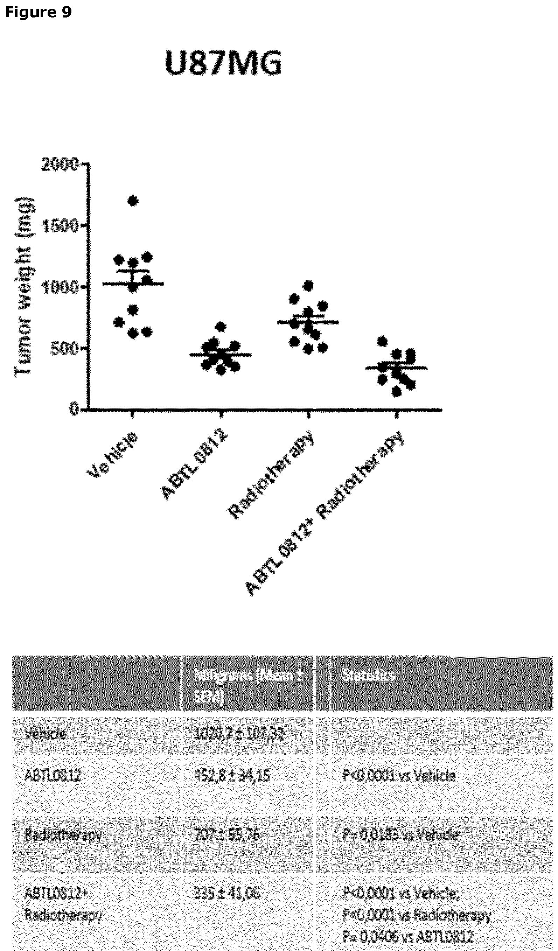

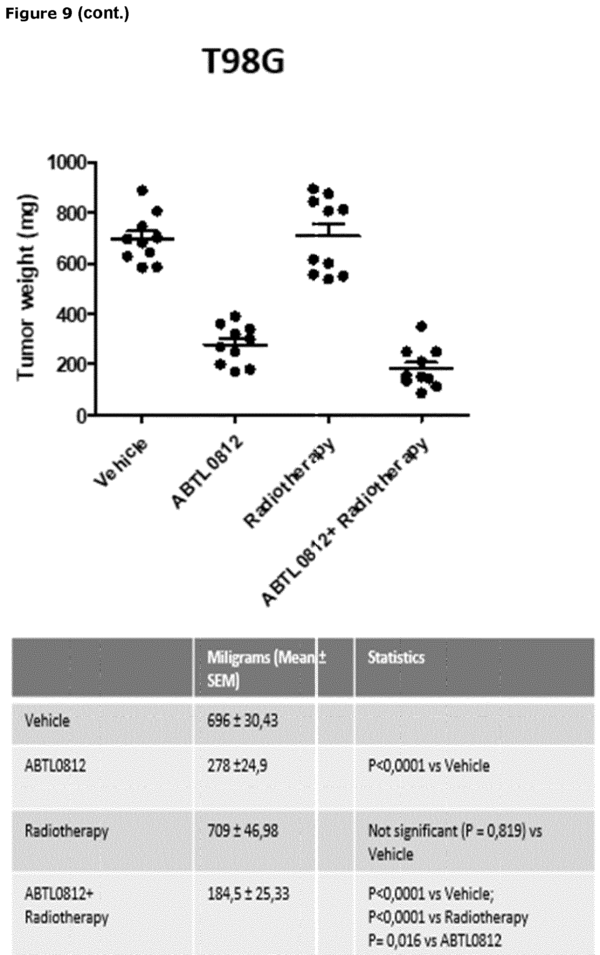

Figure 9: ABTL0812 decreases the growth of glioblastoma tumors (U87MG, T98G cells) and potentiates the antitumor activity of radiotherapy. See working Example herein for further details.

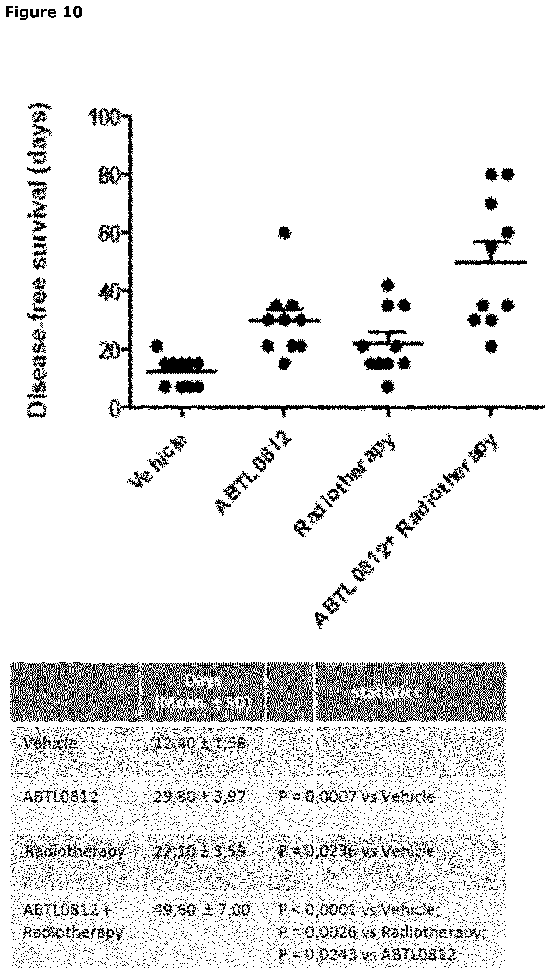

Figure 10: ABTL0812 increases the disease-free survival of mice bearing glioblastoma tumors and potentiates the antitumor activity of radiotherapy. See working Example herein for further details.

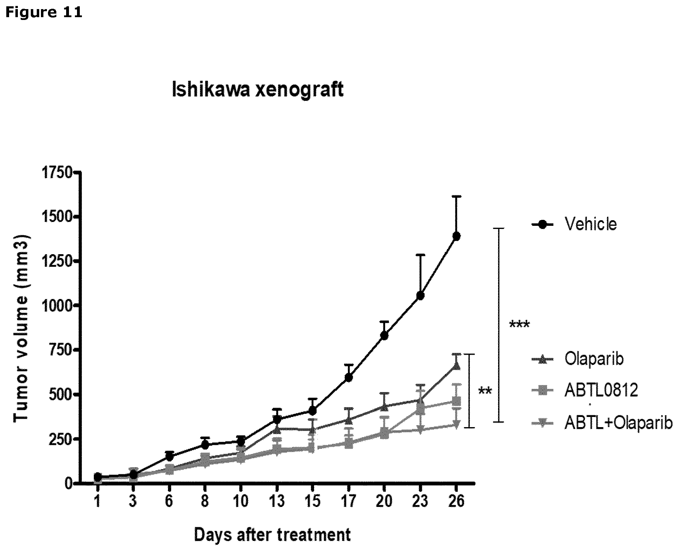

Figure 11: ABTL0812 significantly potentiates Olaparib anticancer effects without increasing toxicity in a human endometrial cancer xenograft model using Ishikawa cells implanted in nude mice. Results suggest a combined therapy of ABTL0812 plus Olaparib could have a clinical interest for the treatment of endometrial cancer. See working Example herein for further details.

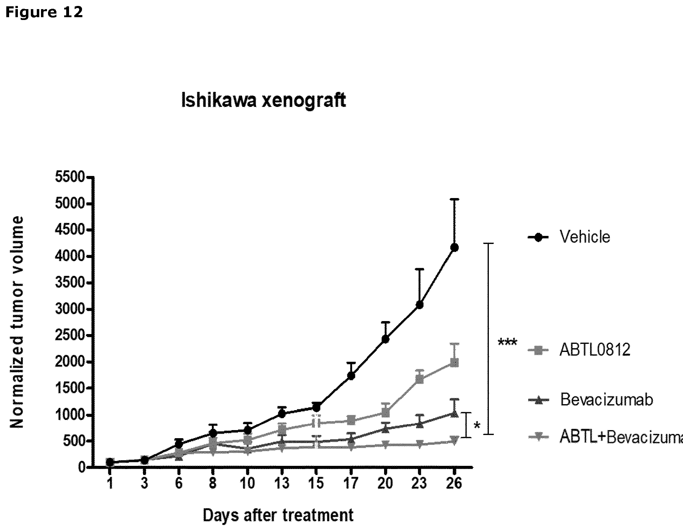

Figure 12: ABTL0812 significantly potentiates bevacizumab anticancer effects without increasing toxicity in a human endometrial cancer xenograft model using Ishikawa cells implanted in nude mice. Results suggest a combined therapy of ABTL0812 plus bevacizumab could have a clinical interest for the treatment of endometrial cancer. See working Example herein for further details.

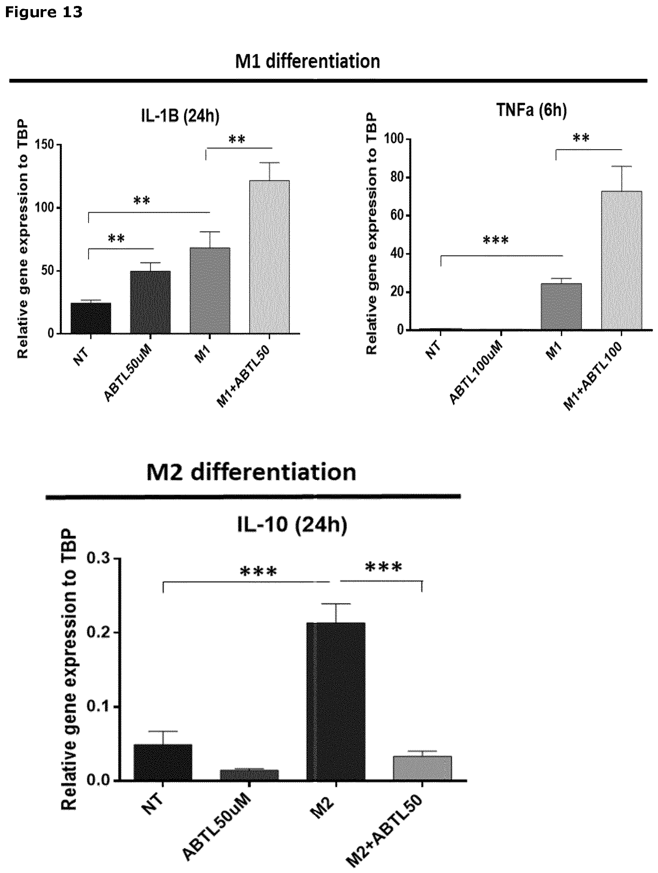

Figure 13: ABTL0812 potentiates macrophage polarization towards a M1 pro-inflammatory anti-tumoral phenotype by significantly increasing IL-1B and TNF-a gene expression. Importantly, ABTL0812 suppresses M2 polarization towards an anti-inflammatory pro-tumoral phenotype by dramatically inhibiting IL10 gene expression, one of the main regulators of immunosuppression. TBP is TATA box binding protein. NT means non polarized macrophages. ABTL50uM means non polarized macrophages treated with 50 µM of ABTL0812. M1 means polarized macrophages to M1 phenotype. M1+ABTL50 means polarized macrophages to M1 phenotype treated with 50 µM of ABTL0812. ABTL100uM means non polarized macrophages treated with 100 µM of ABTL0812. M1+ABTL100 means polarized macrophages to M1 phenotype treated with 100 µM of ABTL0812. M2 means polarized macrophages to M2 phenotype. M2+ABTL50 means polarized macrophages to M2 phenotype treated with 50 µM of ABTL0812. See working Example herein for further details.

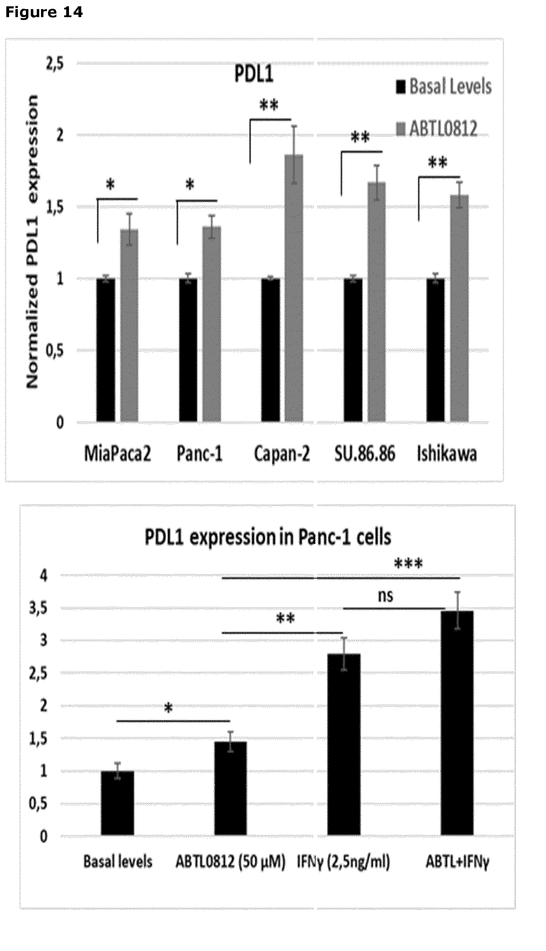

Figure 14: ABTL0812 induces PDL1 expression in endometrial and pancreatic cancer cell lines, ABTL0812-mediated PDL1 expression is significantly inferior to PDL1 expression levels induced by IFNy, master regulator of PDL1 expression. These results highlight the potential combination of ABTL0812 with immune checkpoint inhibitors, since the induction of PDL1 levels mediated will make cancer cells targetable for immune checkpoint inhibitors. See working Example herein for further details.

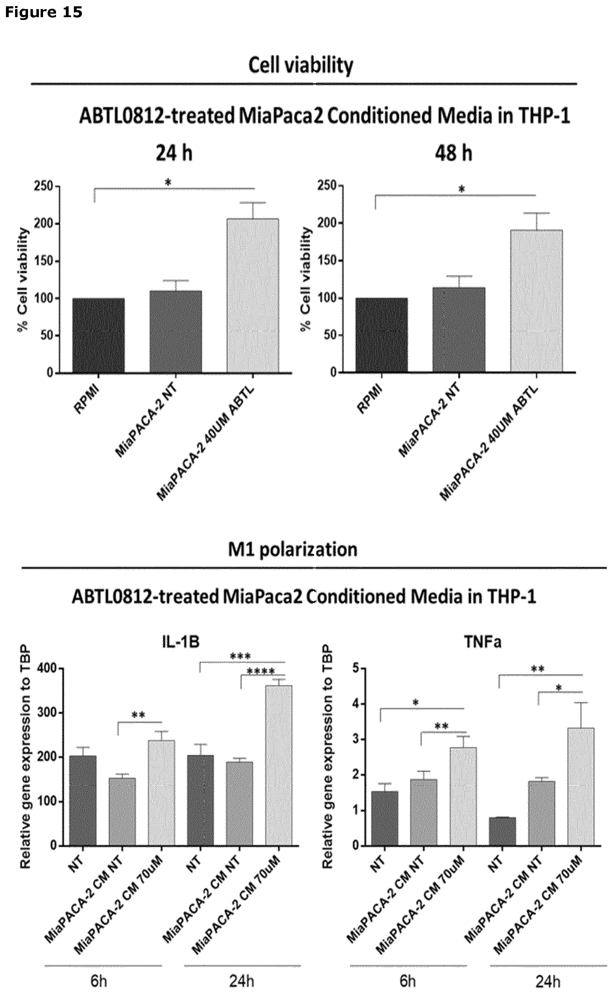

Figure 15: ABTL0812 promotes immunogenic cancer cell death in human pancreatic MiaPaca2 cancer cells, which induces the release of immunogenic factors that promote the sustained activation of macrophages along with their polarization to M1 pro-inflammatory and anti-tumoral phenotypes by significantly inducing a sustained IL-1b and TNF-a sustained gene expression. These data, along with Figure 13, suggest that ABTL0812 is able to immunomodulate the tumor microenvironment by its anticancer effect on cancer cells in addition to its direct effect on human macrophages, therefore highlighting its potential combination with immune checkpoint inhibitors to potentiate the anticancer efficacy. RPMI or NT relates to the initial conditioned media (control). MiaPACA-2 NT or MiaPACA-2 CM NT relates to the conditioned media from non-treated MiaPaca2 cells. MiaPACA-2 40UM ABTL relates to the conditioned media from ABTL0812-treated MiaPaca2 cells (40 µM ABTL0812). MiaPACA-2 CM 70uM relates to the conditioned media from ABTL0812-treated MiaPaca2 cells (70 µM ABTL0812). TBP is TATA box binding protein. See working Example herein for further details.

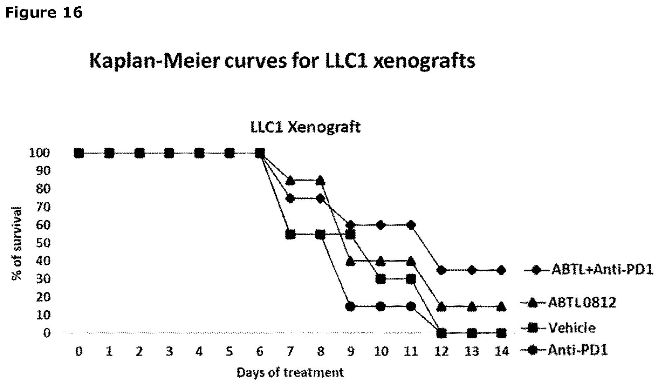

Figure 16: ABTL0812 administered alone increases survival in a syngeneic murine model of lung cancer by LLC1 cells subcutaneously implanted in C57BL6 mice. ABTL0812 in combination with anti-PD1 showed the higher increase in survival, compared with anti-PD1, ABTL0812 and vehicle treatments. Data suggest a potentiation of the anti-PD1 treatment by ABTL0812, leading to an increase in mice survival. This result indicates the potential benefit of combining anti-PD1 and ABTI0812 in human patients. See working Example herein for further details.

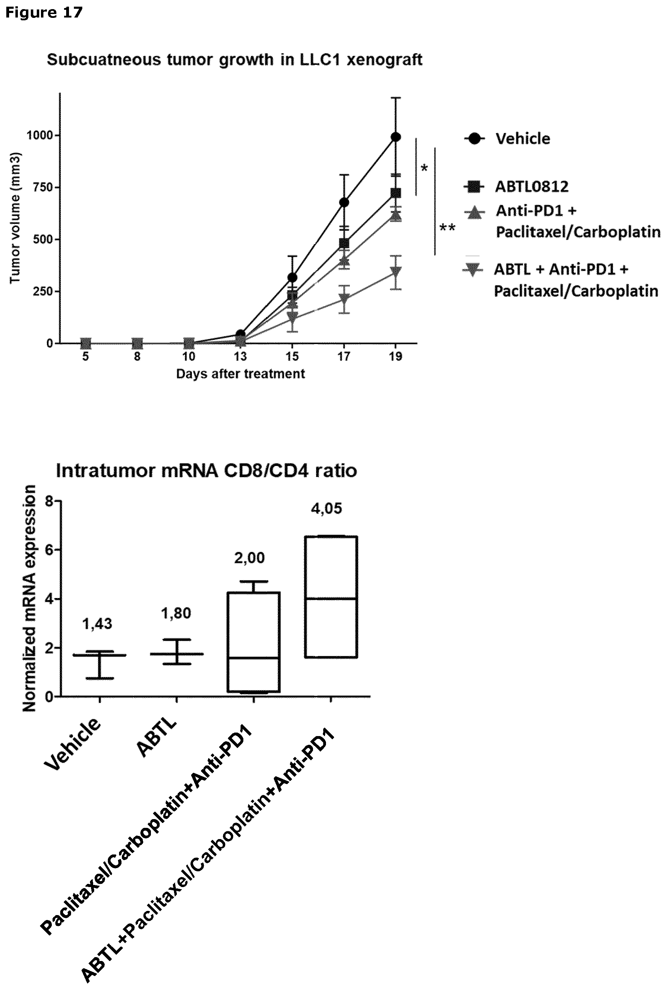

Figure 17: ABTL0812 administered alone shows similar tumor volume reduction as anti-PD1 plus carboplatin/paclitaxel treatment, both treatments significantly reducing tumor volume compared to vehicle group syngeneic murine model of lung cancer by LLC1 cells subcutaneously implanted in C57BL6 mice. The triple combination ABTL0812 plus anti-PD1 plus carboplatin/paclitaxel treatment induces the highest tumor volume reduction, significantly improving the rest of the treatments. This higher anti-cancer efficacy correlated with an increase in CD8/CD4 gene expression levels within tumors, validating *in vivo* previous *in vitro* observations (Figures 15 and 13). CD8/CD4 ration is commonly assessed to analyze cytotoxic anti-tumoral T lymphocytes upon drug treatment. Results suggest a combined therapy of ABTL0812 plus anti-PD1+paclitaxel/carboplatin, a standard treatment for lung cancer patients, could have a clinical interest for the treatment of lung cancer. See working Example herein for further details.

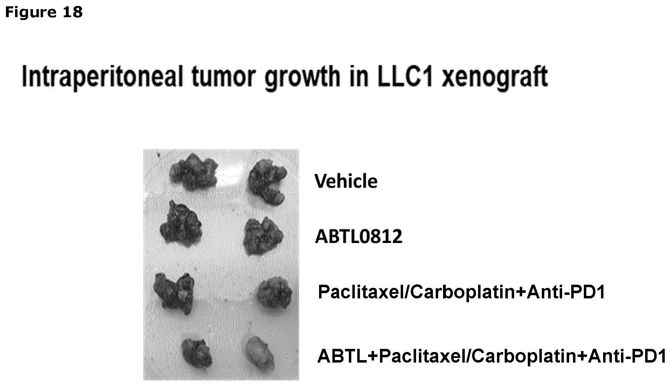

Figure 18: ABTL0812 in combination with anti-PD1 plus carboplatin/paclitaxel treatment induced the highest tumor volume reduction in syngeneic murine model of lung cancer by LLC1 cells intra-peritoneally implanted in C57BL6 mice. Results suggest a combined therapy of ABTL0812 plus anti-PD1+paclitaxel/carboplatin, a standard treatment for lung cancer patients, could have a clinical interest for the treatment of lung cancer. See working Example herein for further details.

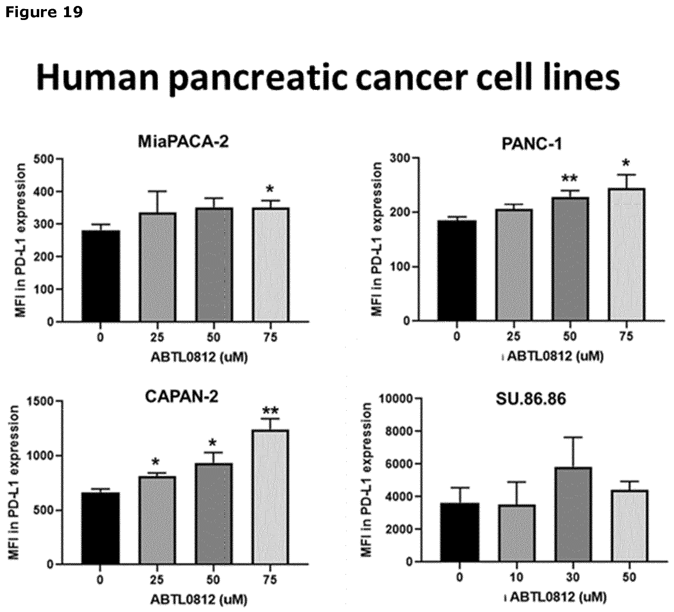

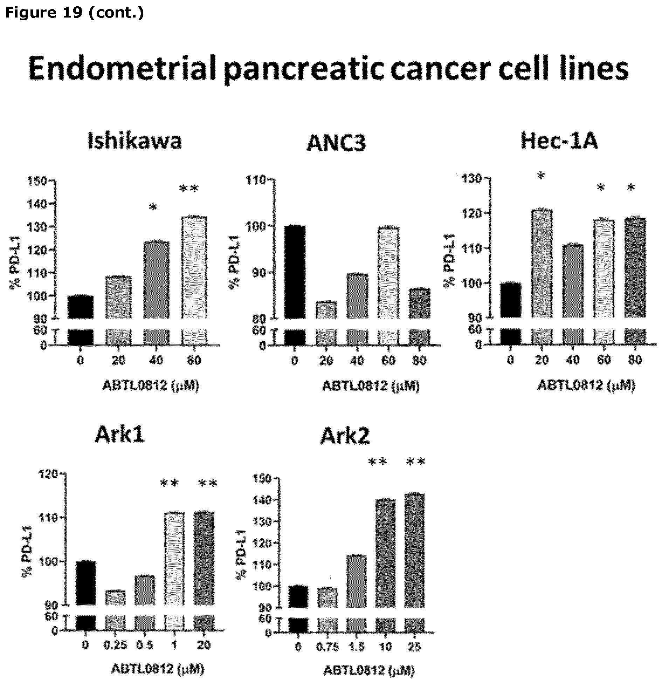

Figure 19: ABTL0812 induces PDL1 expression in endometrial and pancreatic cancer cell lines *in vitro.* These results highlight the potential combination of ABTL0812 with immune checkpoint inhibitors, since the induction of PDL1 levels will make cancer cells targetable for immune checkpoint inhibitors. See working Example herein for further details.

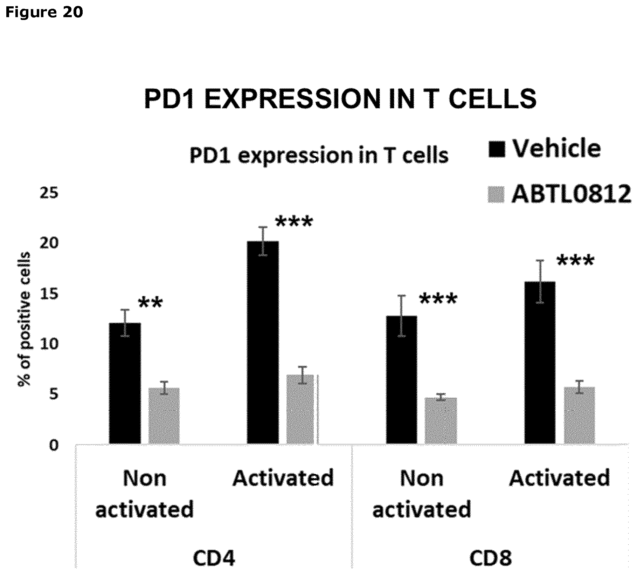

Figure 20: ABTL0812 inhibits the expression of PD1 in activated and non-activated human primary T cells. PD1 mediates an inhibitory signal of T cell activity, thus its reduction by ABTL0812 might facilitate the activation of T cells to elicit its anticancer activity. RFU means relative fluorescence units. WB means Western Blot. See working Example herein for further details.

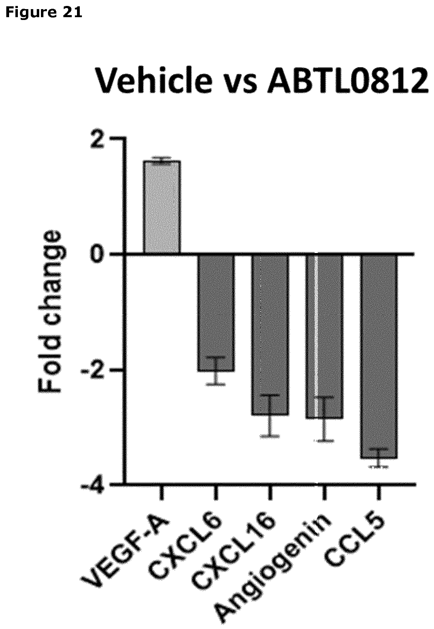

Figure 21: ABTL0812 promotes the inhibition of the release of immunosuppressive chemokines in human cancer cells, which leads to the promotion of a pro-inflammatory environment. See working Example herein for further details.

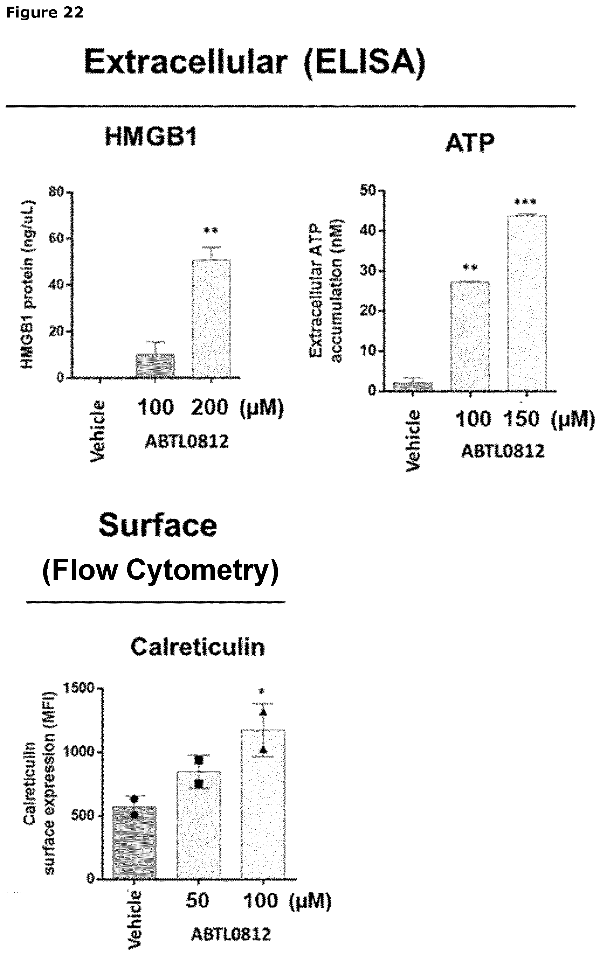

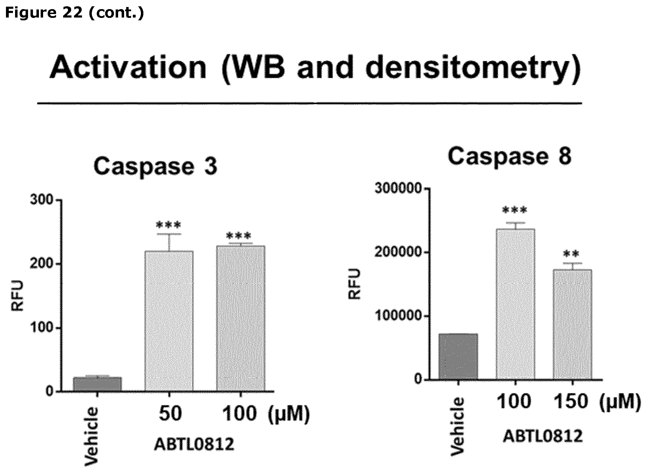

Figure 22: ABTL0812 induces immunogenic cell death (ICD) in human pancreatic cancer cells by inducing a dose-dependent increase in the ICD markers extracellular Hmgb1 and ATP, surface calreticulin and activated caspases 3 and 8. See working Example herein for further details.

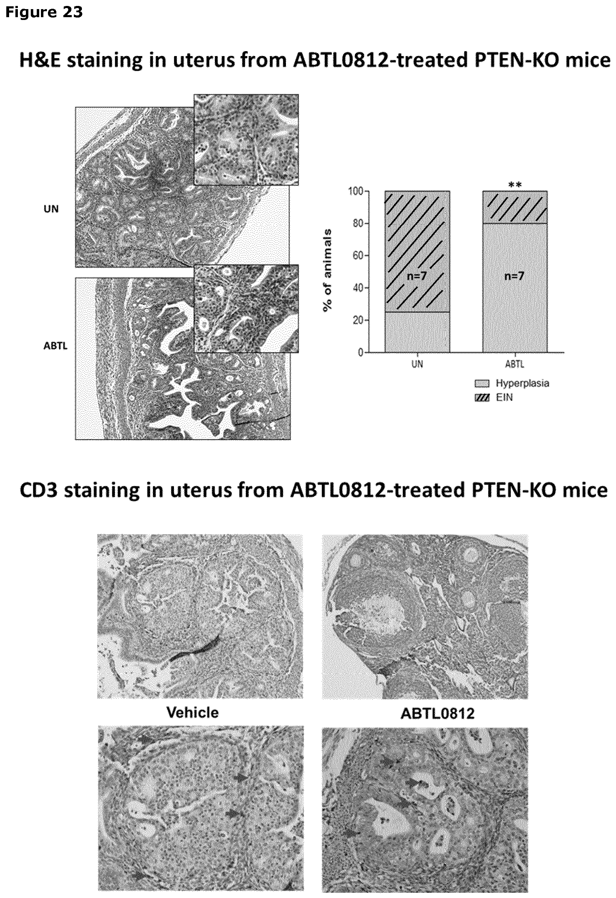

Figure 23: ABTL0812 promotes the infiltration of CD3 T cells within tumoral lesions of PTEN-KO mice undergoing endometrial carcinogenesis, accompanied by a hampering of carcinogenic progression and a decrease in neoplastic lesions (EIN, endometrial intraepithelial neoplasia). UN is untreated.

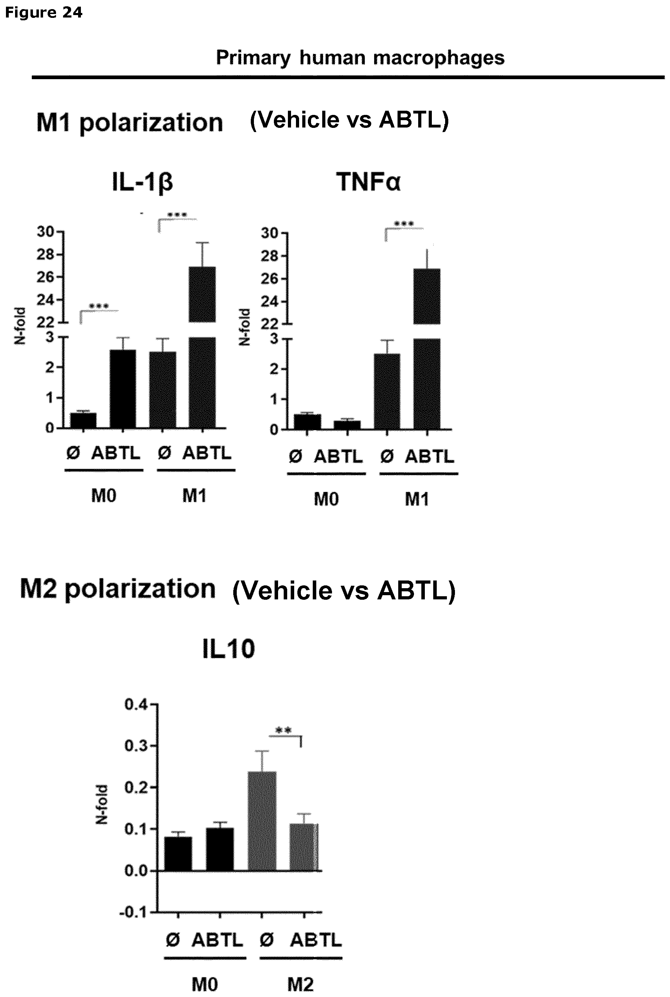

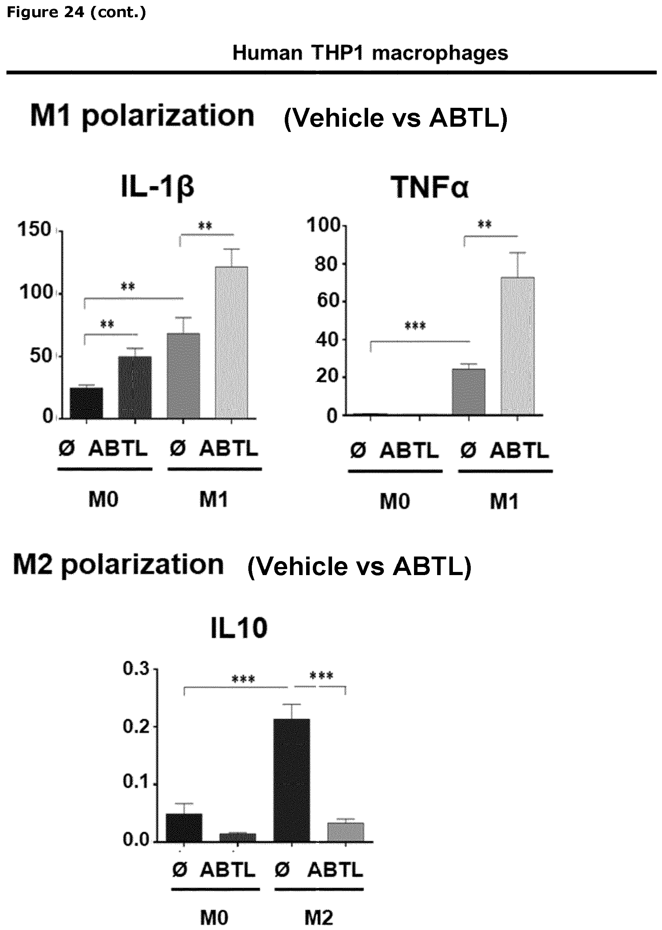

Figure 24: ABTL0812 potentiates immortalized THP-1 and human primary macrophage polarization towards a M1 pro-inflammatory anti-tumoral phenotype by significantly increasing IL-1ß and TNF-α gene expression. Importantly, ABTL0812 suppresses M2 polarization towards an anti-inflammatory pro-tumoral phenotype by dramatically inhibiting IL10 gene expression, one of the main regulators of immunosuppression. These data suggest an immunomodulatory effect of ABTL0812 in macrophages towards an anti-tumor phenotype, potentially synergizing with immunotherapy. ø means vehicle. See working Example herein for further details.

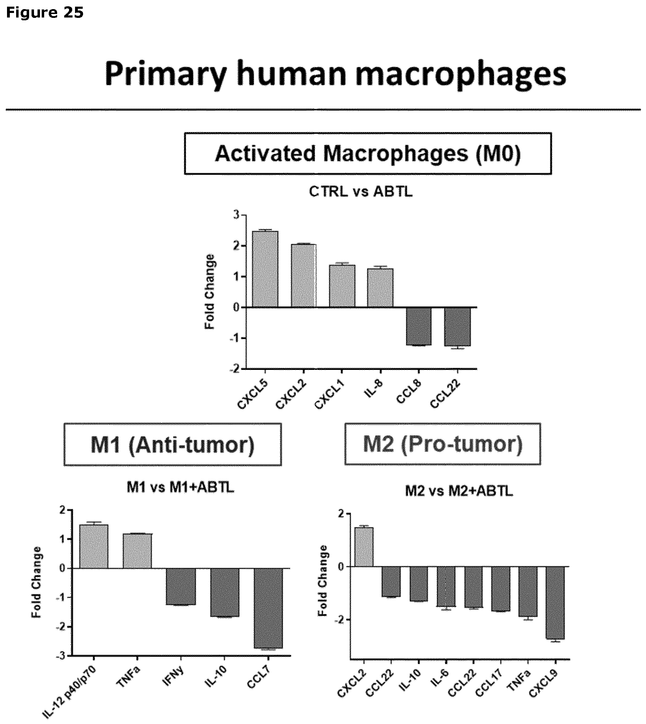

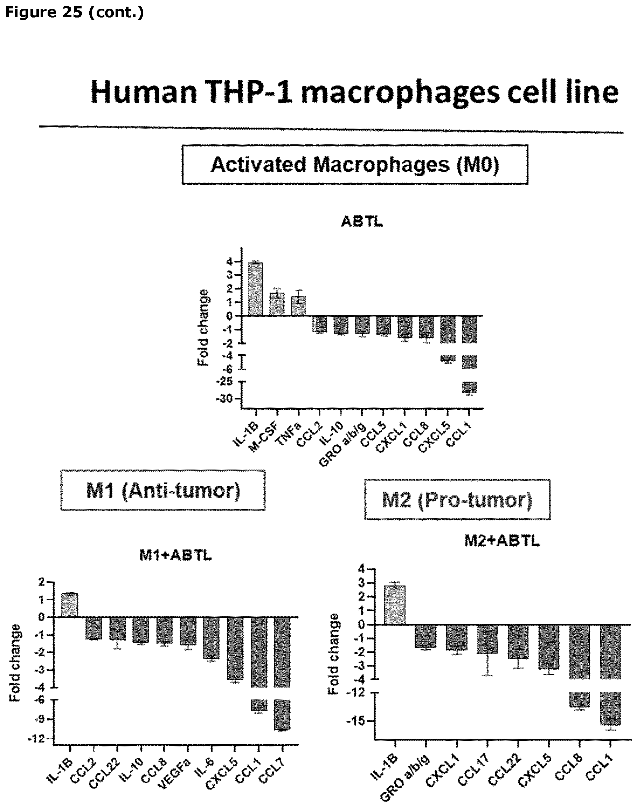

Figure 25: ABTL0812 promotes release of proinflammatory cytokines and the inhibition of the secretion of immunosuppressive cytokines in human primary macrophages and in immortalized THP1 macrophages, which leads to the promotion of a pro-inflammatory environment. These results support a potential synergy with immunotherapy. CTRL means control. See working Example herein for further details.

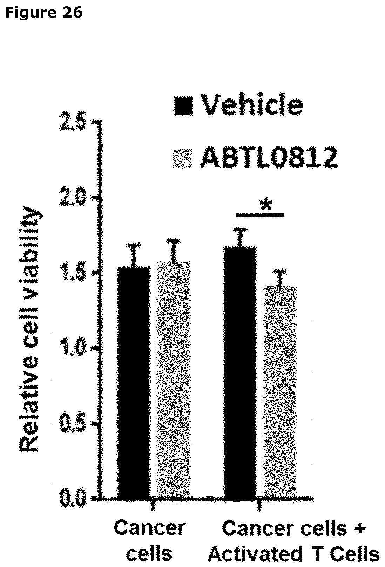

Figure 26: ABTL0812 potentiates the cytotoxic effect of activated T cells against cancer cells when both cell types are co-cultured *in vitro.*

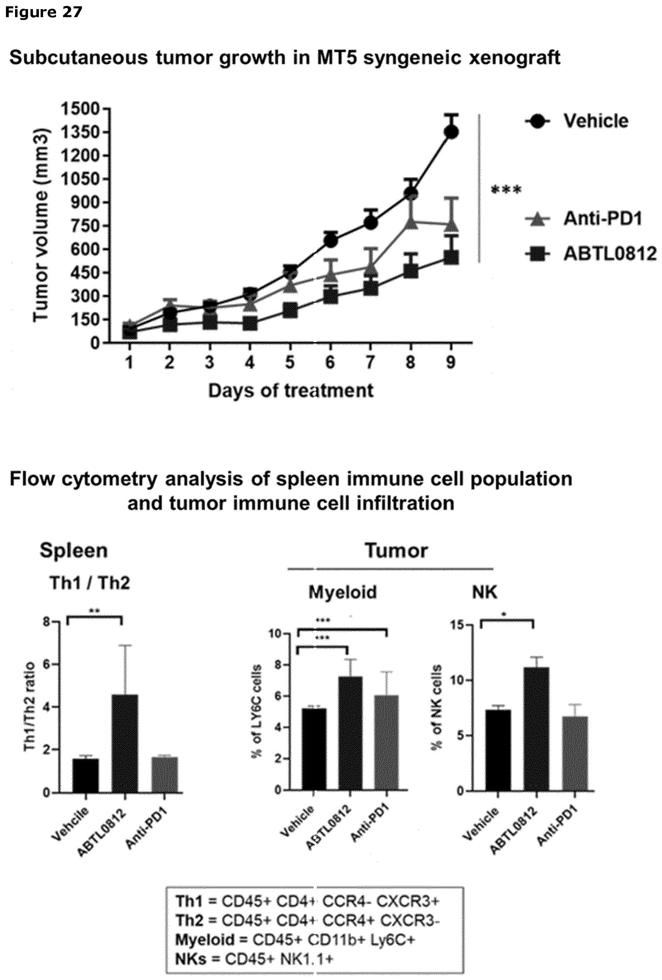

Figure 27: ABTL0812 monotherapy shows similar tumor volume reduction as anti-PD1, both treatments significantly reducing tumor volume compared to vehicle group in a syngeneic murine model of MT5 pancreatic cancer cells subcutaneously implanted in C57BL6 mice. ABTL0812 promotes a pro-inflammatory antitumor environment more efficiently than anti-PD1 treatment, leading to an increase in myeloid and NK anticancer cells within tumors, and an increase in the Th1/Th2 ration in spleens.

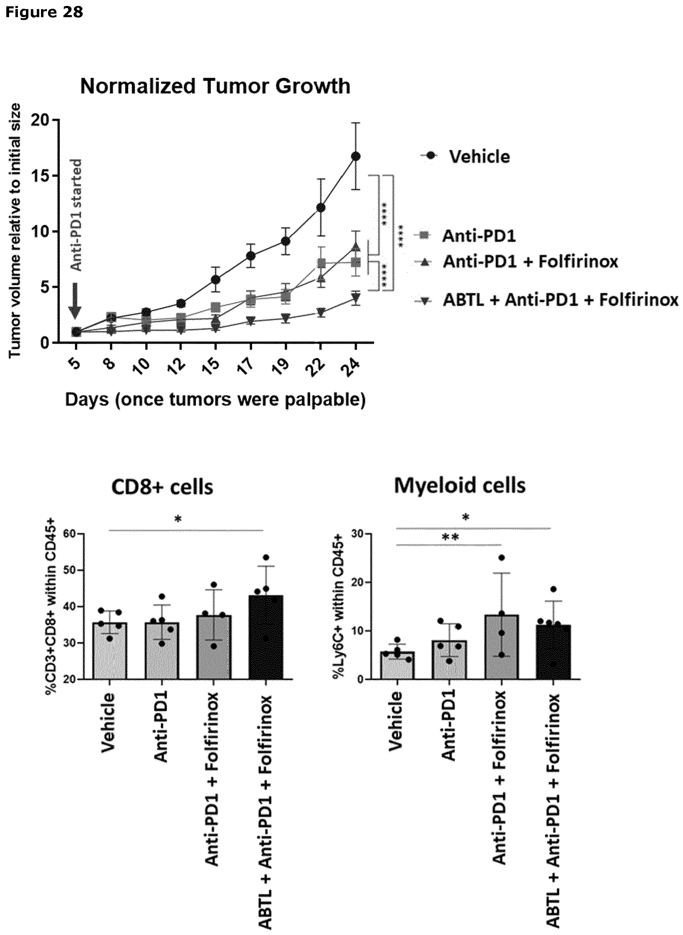

Figure 28: ABTL0812 administered in combination with anti-PD1 and FOLFIRINOX shows the highest tumor volume reduction compared with the rest of the groups, including anti-PD1 alone and ABTL0812 + FOLFIRINOX, promoting a pro-inflammatory antitumor environment with increased myeloid and CD8 anticancer cells infiltration within tumors.

### DETAILED DESCRIPTION OF THE INVENTION

### Compound (A) of the relevant aspects

Compound (A) is COOH-CHOH-(CH₂)₆-(CH=CH-CH₂)₂-(CH₂)₃-CH₃ (ABTL0812) or a pharmaceutically acceptable salt thereof.

A pharmaceutically acceptable salt of Compound (A) refers to any pharmaceutically acceptable salt of Compound (A). As known in the art, there are numerous known pharmaceutically acceptable salts. Examples of pharmaceutically acceptable salts include, but are not limited to, sodium (Na), potassium, acetates, sulfates, pyrosulfates, bisulfates, sulfites, bisulfites, phosphates, monohy-drogenphosphates, dihydrogenphosphates, metaphosphates, pyrophosphates, chlorides, bromides, iodides, acetates, propionates, decanoates, caprylates, acrylates, formales, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phylacetates, phenylpropionates, phenylbutyrates, citrates, lactates, gamma-hydroxybutyrates, glycollates, tartarates, alkanesulfonates (e.g. methane-sulfonate or mesylate), propanesulfonates, naphthalene-1-sulfonates, naphthalene-2-sulfonates, and mandelates. In a particular embodiment, the salt of Compound (A) is the sodium salt.

As understood by the skilled person in the present context, when there herein is referred to a preferred formula of Compound (A), such as e.g. ABTL0812 - it is herein understood that it also included as salt thereof - for instance, when there herein is referred to that Compound (A) is COOH-CHOH-(CH₂)₆-(CH=CH-CH₂)₂-(CH₂)₃-CH₃ (ABTL0812) then there is also referred to a salt of ABTL0812.

Preferably, Compound (A) is a sodium salt of COOH-CHOH-(CH₂)₆-(CH=CH-CH₂)₂-(CH₂)₃-CH₃ (ABTL0812).

### Immunotherapy agent - Compound (B3)

As known in the art, checkpoint inhibitor therapy is a form of cancer immunotherapy. The therapy targets immune checkpoints, key regulators of the immune system that when stimulated can dampen the immune response to an immunologic stimulus. Checkpoint inhibitors are molecules capable of blocking immune checkpoint proteins. Consequently, checkpoint inhibitors enhance immune responses promoting elimination of cancer cells.

Currently approved checkpoint inhibitors are generally antibodies and target the molecules CTLA4, PD-1, and PD-L1 - these checkpoint inhibitors may be termed anti-PD1, anti-PDL1, anti-CTLA4 checkpoint inhibitors.

The Conclusion of working Example 6.1 below reads:
"These results suggest that ABTL0812, apart from its anti-cancer effect on tumor cells, stimulates immune system to a pro-inflammatory phenotype, altering tumor microenvironment promoting the recruitment of other immune cells as cytotoxic T lymphocytes, thus making a "cold" tumor which induces immune system suppression, to a "hot" and immunogenic tumor, highlighting the potential combination of ABTL0812 with in particular immune checkpoint inhibitors to potentiate anticancer efficacy by promoting a pro-inflammatory and anti-tumor microenvironment."

The Conclusion of working Example 7 below reads:
"... ABTL0812 immunomodulatory effects *in vivo* show how it induces the infiltration of T lymphocytes within tumoral lesions, indicative of the presence of a pro-inflammatory anti-tumoral microenvironment that favors the infiltration of immune cells to kill cancer cells.... highlighting its potential combination with in particular immune checkpoint inhibitors to potentiate anticancer efficacy."

Accordingly, experimental data herein (see Examples 6-8) provides evidence for that it is plausible that ABTL0812 as such has positive immunomodulatory effects in relation to immunotherapy treatment as such of a cancer - in particular in relation to use of immune checkpoint inhibitors.

Tumors can manipulate PD-1/PD-L1 immune checkpoint pathway to shut down cancer-targeting T-cells. Thus, in some embodiments, Compound (B3) is a checkpoint inhibitor targeting PD-1/PD-L1 pathway, also named anti PD-1 or anti PD-L1, that can enable T cells to eliminate cancer cells.

CTLA-4 is another pathway that can be targeted by checkpoint inhibitors capable of blocking CTLA-4 receptor.

In some embodiments, checkpoint inhibitors can be antibody-based agents or non-antibody-based agents (e.g. small molecules or peptides).

The checkpoint inhibitor is a checkpoint inhibitor antibody. Examples of checkpoint inhibitors targeting PD-1/PDL-1 pathway are Atezolizumab, Avelumab, Cemiplimab, Durvalumab, Nivolumab and Pembrolizumab. Ipilimumab is an example of a checkpoint inhibitor which targets CTLA-4 pathway.

Compound (B3) is at least one immunotherapy agent compound which is a checkpoint inhibitor selected from the group consisting of:
- an anti-PD1 antibody, particularly wherein the anti-PD1 antibody is Nivolumab, Pembrolizumab or Spartalizumab; and
- an anti-PDL1 antibody, particularly wherein the anti-PDL1 antibody is Atezolizumab, Avelumab or Durvalumab.

As understood in the present context - in relation to any of the preferred listed examples of Compound (B3) is it most preferred that Compound (A) is COOH-CHOH-(CH₂)₆-(CH=CH-CH₂)₂-(CH₂)₃-CH₃ (ABTL0812).

Examples of preferred anti-PD1 checkpoint inhibitor antibodies are Nivolumab, Pembrolizumab or Spartalizumab.

In working Example herein was obtained positive results with an anti-PD1 checkpoint inhibitor antibody that may be seen as corresponding to Pembrolizumab. In short, Pembrolizumab is for human use and in working Example herein was used a modified version optimized for use in the murine model used in working Example herein.

Accordingly, in a preferred embodiment is the anti-PD1 checkpoint inhibitor antibody Pembrolizumab.

Examples of preferred anti-PDL1 checkpoint inhibitor antibodies are Atezolizumab, Avelumab or Durvalumab.

An example an anti-CTLA4 checkpoint inhibitor antibody is lpilimumab.

Example 8 herein shows positive results in relation to use of an anti-PD1 checkpoint inhibitor antibody - accordingly, a preferred embodiment relates to wherein Compound (B3) is an anti-PD1 checkpoint inhibitor antibody (preferably Nivolumab, Pembrolizumab or Spartalizumab) - in particular wherein Compound (A) is ABTL0812.

It may be preferred that Compound (B3) of the aspect comprises two or more different checkpoint inhibitor antibodies (in particular when Compound (A) is COOH-CHOH-(CH₂)₆-(CH=CH-CH₂)₂-(CH₂)₃-CH₃ (ABTL0812)).

As understood by the skilled person in the present context - one may combine use of Compound (B3) with other cancer treatment relevant agents/compounds, such as e.g. one or more chemotherapeutic agent compound(s).

It is particular preferred that Compound (A) is ABTL0812 and Compound (B3) is an anti-PD1 checkpoint inhibitor antibody (most preferably Pembrolizumab) - in particular wherein the cancer is lung cancer (See Example 8.1 for an example of this preferred embodiment).

It is particular preferred that Compound (A) is ABTL0812 and Compound (B3) is an anti-PD1 checkpoint inhibitor antibody (most preferably Pembrolizumab), which are further administered in combination with at least one Compound (B1), e.g. Paclitaxel and Carboplatin - in particular wherein the cancer is lung cancer (See Examples 8.2 and 8.3 for an example of this preferred embodiment).

Preferably, the pharmaceutical combination as discussed herein is wherein Compound (A) is ABTL0812 and wherein:
- Compound (B3) is an anti-PD1 checkpoint inhibitor antibody (most preferably Pembrolizumab) and the cancer is lung cancer; or
- Compound (B3) is an anti-PD1 checkpoint inhibitor antibody (most preferably Pembrolizumab), which is administered in combination with at least one Compound (B1), particularly, Paclitaxel and Carboplatin, and the cancer is lung cancer.

The Conclusion of working Example 6.7 reads below (emphasis added):
"ABTL0812 could induce ICD (immunogenic cell death) in tumors, making them more immunogenic and targetable for the immune system, helping make a "cold" tumor which induces immune system suppression, to a "hot" and immunogenic tumor ..."

The Conclusion of working Example 6.9 reads below (emphasis added):
"ABTL0812 promotes the secretion of pro-inflammatory factors and suppresses the release of immunosuppressive factors in cancer cells. These data, in combination with the potentiation of M1 macrophage phenotype and the suppression of M2 phenotypes suggest that ABTL0812 can promote a pro-inflammatory anti-tumor environment towards its action on the immune cells, highlighting its potential combination with other immunotherapies to increase the efficacy against tumors, particularly in those highly immunosuppressive, such as pancreatic cancers."

Accordingly, experimental data herein (see Examples 6-8) provides evidence for that it is plausible that ABTL0812 as such has positive immunomodulatory effects to enhance the anticancer efficacy of treatments that interact with immune system response and its regulation (e.g. cytokines) other than checkpoint inhibitors. Thus, it is evident to the skilled person that ABTL0812 can potentiate the anticancer effect of immunomodulators.

Thus, in some embodiments, Compound (B3) is an anticancer immunomodulatory agent compound. The term "anticancer immunomodulatory agent compound" (also named as "anticancer immunomodulator agent compound") is used herein to refer to immunomodulators as molecules able to target pathways that regulate the immune system's activity, improving its ability to attack and eliminate cancer cells. Immunomodulators is a known group of molecules within cancer immunotherapy agents, and can comprise e.g. checkpoint inhibitors, cytokines, agonists and adjuvants. The regulation of the immune system comprises the stimulation or inhibition of immune system's mechanisms.

In some embodiments, Compound (B3) is an immunotherapy agent compound which is an anti-cancer immunomodulatory agent compound, which is a cytokine. Cytokines are messenger molecules that regulate immune cell maturation, growth and responsiveness. Examples of immunomodulator cytokines are cytokines targeting IL-2/IL-2R pathway and cytokines targeting IFNAR1 and/or IFNAR2 pathways. Aldesleukin (Proleukin^{®}) is an example of an immunomodulator cytokine targeting IL-2/IL-2R pathway. Examples of immunomodulator cytokines targeting IFNAR1 and/or IFNAR2 pathways are Interferon alfa-2a, Interferon alfa-2b (Intron A^{®}), and Peginterferon alfa-2b (Syla-tron^{®}/PEG-Intron^{®}). Other examples of cytokines are Granulocyte-macrophage colony-stimulating factor (GM-CSF) for the treatment of neuroblastoma.

In some embodiments, Compound (B3) is an immunotherapy agent compound which is an anti-cancer immunomodulatory agent compound, which is an agonist. Agonists are molecules capable of activating pathways that promote adaptive immune responses. For instance, immunomodulator agonists can enhance activation of "killer" T cells or stimulate the activity of innate immunce cells (e.g. dendritic cells).

In some embodiments, Compound (B3) is an immunotherapy agent compound which is an anti-cancer immunomodulatory agent compound, which is an adjvant. Adjuvants are molecules capable of activating pathways involved in the innate immune system which can stimulate general immune responses and ultimately promote adaptive immune resonses. Examples of immunomodulator adjuvants are those targeting toll-like receptors (e.g. TLR7 or TLR3). Imiquimod and Poly ICLC (Hiltonol^{®}) are examples of adjuvants targeting toll like receptors for the treatment of cancer.

Compound (A) (in particular ABTL0812) is preferably administrated orally.

The administrated dose of Compound (A) (in particular ABTL0812) is preferably a daily dose of from 200 mg to 7000 mg, more preferably a daily dose of from 1500 mg to 5000 mg, even more preferably a daily dose of from 3000 mg to 4700 mg and most preferably a daily dose of from 3500 mg to 4300 mg.

Preferably, the daily dose of Compound (A) (in particular ABTL0812) is a daily dose administrated 3 times per day - most preferably as 3 times 1200-1400 mg.

### Other particular combinations

In working Examples herein were obtained positive results with a combination of Compound (A), a Compound (B1) and a Compound (B3), i.e. with a triple combination.

Accordingly, the invention also relates to a pharmaceutical combination comprising:
(A): a compound which is a polyunsaturated fatty acid of formula COO**R₁**-CH**R₂**-(CH₂)**a-**(CH=CHCH₂)**b**-(CH₂)**c**-CH₃, a pharmaceutically acceptable salt thereof, or a combination thereof, wherein
   (i) **a** can be any integer value between 0 and 7,
   (ii) **b** can be any integer value between 2 and 7,
   (iii) **c** can be any integer value between 0 to 7,
   (iv) **R₁** is H, Na, K, CH₃, CH₃-CH₂, or PO(O-CH₂-CH₃)₂, and
   (v) **R₂** is OH, OCH₃, O-CH₂COOH, CH₃, Cl, CH₂OH, OPO(O-CH₂-CH₃)₂, N(OH)₂, F, HCOO or N(OCH₂CH₃)₂;
(B1): a chemotherapeutic agent compound; and
(B3): an immunotherapy agent compound
for the simultaneous, separate or sequential use in the treatment of a cancer in a human patient.

In a particular embodiment: (i) **a** can be any integer value between 5 and 7, (ii) **b** can be any integer value between 2 and 4, and (iii) **c** can be any integer value between 1 to 5. In another embodiment, **R₁** is H and **R₂** is OH.

In a particular embodiment, Compound (A) is at least one compound or a pharmaceutically acceptable salt thereof selected from the group consisting of:

COOH-CHOH-(CH₂)₆-(CH=CH-CH₂)₂-(CH₂)₃-CH₃ (ABTL0812),

COOH-CHOH-(CH₂)₆-(CH=CH-CH₂)₃-CH₃ (183A1),

COOH-CHOH-(CH₂)₃-(CH=CH-CH₂)₃-(CH₂)₃-CH₃ (183A2),

COOH-CHOH-(CH₂)₂-(CH=CH-CH₂)₄-(CH₂)₃-CH₃ (204A1),

COOH-CHOH-(CH₂)₂-(CH=CH-CH₂)₅-CH₃ (205A1)

and

COOH-CHOH-CH₂-(CH=CH-CH₂)₆-CH₃ (226A1).

Particularly, Compound (A) is COOH-CHOH-(CH₂)₆-(CH=CH-CH₂)₂-(CH₂)₃-CH₃ (ABTL0812) or a pharmaceutically acceptable salt thereof. More particularly, Compound (A) is a sodium salt of COOH-CHOH-(CH₂)₆-(CH=CH-CH₂)₂-(CH₂)₃-CH₃ (ABTL0812).

In some embodiments, the cancer is at least one cancer selected from the group consisting of:
Lung cancer;
Non-small cell lung cancer;
Squamous cell cancer;
Adenocarcinoma;
Endometrial cancer;
Endometrial serous cancer;
Endometroid cancer;
Pancreatic cancer;
Glioblastoma;
Resistant-recurrent breast cancer;
Head and neck cancer;
Multiple myeloma cancer;
Neuroblastoma and
Cholangiocarcinoma.

In some embodiments, Compound (B3) is an immunotherapy agent compound which is an anti-cancer immunomodulatory agent compound. In a particular embodiment, Compound (B3) is a checkpoint inhibitor. Particular embodiments and examples are described in section of this description "Immunotherapy agent - Compound (B3)".

In some embodiments, Compound (B3) is at least one immunotherapy agent compound selected from the group consisting of: Checkpoint inhibitor, preferably checkpoint inhibitor antibody. Preferably, the checkpoint inhibitor antibody is an anti-PD1 antibody, an anti-PDL1 antibody or an anti-CTLA4 antibody.

In some embodiments, Compound (B3) is at least one immunotherapy agent compound selected from the group consisting of:
- anti-PD1 antibody, preferably wherein the anti-PD1 antibody is Nivolumab, Pembrolizumab or Spartalizumab;
- anti-PDL1 antibody, preferably wherein the anti-PDL1 antibody is Atezolizumab, Avelumab or Durvalumab;
- anti-CTLA4 antibody, preferably wherein the anti-CTLA4 antibody is lpilimumab.

Particularly, Compound (B3) is an anti-PD1 antibody, preferably wherein the anti-PD1 antibody is Pembrolizumab.

In some embodiments:
- Compound (B3) is an anti-PD1 checkpoint inhibitor antibody, preferably Pembrolizumab, and the cancer is lung cancer; or
- Compound (B3) is an anti-PD1 checkpoint inhibitor antibody, preferably Pembrolizumab, which is administered in combination with at least one Compound (B1), preferably Paclitaxel and Carboplatin and the cancer is lung cancer.

In some embodiments, Compound (B1) is at least one chemotherapeutic agent compound selected from the group consisting of:
Temozolomide;
Topotecan;
Irinotecan;
Cyclophosphamide;
Fluorouracil;
Cisplatin;
Carboplatin;
Oxaliplatin;
Leucovorin;
Doxorubicin;
Bleomycin;
Capecitabine;
Mitomycin B;
Paclitaxel;
Nab-paclitaxel;
Docetaxel;
Gemcitabine;
Methotrexate;
Pemetrexed;
5- Fluorouracil;
Cytarabine;
Mercaptopurine;
Glufosfamide;
Ixabepilone;
Nimustine;
Carmustine;
Lomustine;
Mitoxantrone;
Etoposide;
Vincristine;
Vinblastine; and
Tamoxifen.

In a particular embodiment, Compound (B1) is at least one chemotherapeutic agent compound selected from the group consisting of:
Temozolomide;
Topotecan;
Irinotecan;
Cyclophosphamide;
Fluorouracil;
Oxaliplatin;
Leucovorin; and
Doxorubicin.

Particularly, Compound (B1) is at least one chemotherapeutic agent compound selected from the group consisting of:
Temozolomide;
Topotecan;
Fluorouracil;
Oxaliplatin; and
Leucovorin.

In another embodiment, Compound (B1) is at least one chemotherapeutic agent compound selected from the group consisting of:
Irinotecan;
Fluorouracil;
Oxaliplatin; and
Leucovorin.

In another embodiment, Compound (B1) is at least one chemotherapeutic agent compound selected from the group consisting of:
Carboplatin; and
Paclitaxel.

In another embodiment, Compound (B1) is at least one chemotherapeutic agent compound selected from the group consisting of:
Temozolomide;
Topotecan;
Irinotecan;
Cyclophosphamide;
Fluorouracil;
Oxaliplatin;
Leucovorin;
Doxorubicin;
Carboplatin; and
Paclitaxel.

Particularly, Compound (B1) is paclitaxel and carboplatin.

In another embodiment, Compound (B1) is irinotecan, leucovorin, oxaliplatin and fluorouracil.

In a particular embodiment, Compound (A) is ABTL0812, Compound (B1) is paclitaxel and carboplatin and Compound (B3) is an anti-PD1 checkpoint inhibitor antibody.

In one embodiment, Compound (A) is ABTL0812, Compound (B1) is irinotecan, leucovorin, oxaliplatin and fluorouracil and Compound (B3) is an anti-PD1 checkpoint inhibitor antibody. Particularly, Compound (B3) is an anti-PD1 checkpoint inhibitor antibody selected from Nivolumab, Pembrolizumab and Spartalizumab, preferably Pembrolizumab.

It is particular preferred that Compound (A) is ABTL0812, Compound (B1) comprises Paclitaxel/Carboplatin and Compound (B3) is an anti-PD1 checkpoint inhibitor antibody. In a particular embodiment, the cancer is lung cancer (See Examples 8.2 and 8.3 for an example of this preferred embodiment).

It is particular preferred that Compound (A) is ABTL0812, Compound (B1) comprises Irinotecan, Leucovorin, Oxaliplatin and Fluorouracil and Compound (B3) is an anti-PD1 checkpoint inhibitor antibody. In a particular embodiment, the cancer is pancreatic cancer (See Example 8.5 for an example of this preferred embodiment).

Depending on the type of Compound (B1), it can be administered e.g. intravenously (e.g. in case of an antibody) or orally (e.g. in case of a small molecule). Particularly, Compound (3) is administrated intravenously via infusion solution.

Particularly, Compound (A) is administrated orally. In a particular embodiment, wherein the administrated dose of Compound (A) is a daily dose of from 200 mg to 7000 mg, more preferably a daily dose of from 1500 mg to 5000 mg, even more preferably a daily dose of from 3000 mg to 4700 mg and most preferably a daily dose of from 3500 mg to 4300 mg.

### A cancer

Preferably, the cancer is at least one cancer selected from the group consisting of:
Lung cancer;
Non-small cell lung cancer;
Squamous cell cancer;
Adenocarcinoma;
Endometrial cancer;
Endometrial serous cancer;
Endometroid cancer;
Pancreatic cancer;
Glioblastoma;
Resistant-recurrent breast cancer;
Head and neck cancer;
Multiple myeloma cancer;
Neuroblastoma and
Cholangiocarcinoma.

More preferably, the cancer is at least one cancer selected from the group consisting of:
Non-small cell lung cancer;
Squamous cell cancer;
Endometrial cancer;
Pancreatic cancer;
Glioblastoma;
Breast cancer;
Multiple myeloma cancer;
Neuroblastoma; and
Cholangiocarcinoma.

More particularly, the cancer is at least one cancer selected from the group consisting of:
Lung cancer;
Endometrial cancer;
Pancreatic cancer;
Glioblastoma;
Breast cancer;
Neuroblastoma; and
Cholangiocarcinoma.

In a particular embodiment, the cancer is a solid tumor. "Solid tumors" or solid cancers are neoplasms (new growth of cells) or lesions (damage of anatomic structures or disturbance of physiological functions) formed by an abnormal growth of body tissue cells other than blood, bone marrow or lymphatic cells. A solid tumor consists of an abnormal mass of cells which may stem from different tissue types such as liver, colon, breast, or lung, and which initially grows in the organ of its cellular origin. However, such cancers may spread to other organs through metastatic tumor growth in advanced stages of the disease.

In particular embodiments, the cancer is a carcinoma, a sarcoma, a germinoma or a blastoma.

In a particular embodiment, the cancer is a carcinoma. Carcinomas are cancers derived from epithelial cells and account for 80% to 90% of all cancer cases since epithelial tissues are most abundantly found in the body. In particular embodiments, the carcinoma includes many of the most common cancers, particularly e.g. lung cancer, colorectal cancer, pancreatic cancer, larynx cancer, tongue cancer, prostate cancer, breast cancer, ovarian cancer, liver cancer, head and neck cancer, esophageal cancer, renal cancer, endometrial cancer, gall bladder cancer, bladder cancer or gastric cancer.

Carcinomas are of two types: adenocarcinoma and squamous cell carcinoma. Adenocarcinoma develops in epithelial cells or a gland and squamous cell carcinoma originates in squamous epithelium. Adenocarcinomas may affect mucus membranes and are first seen as a thickened plaque-like white mucosa. These are rapidly spreading cancers.

In a particular embodiment, the cancer is adenocarcinoma.

In particular embodiments, the cancer is lung cancer, endometrial cancer, or pancreatic cancer.

In a particular embodiment, the cancer is lung cancer, more particularly, non-small cell lung cancer.

In a particular embodiment, the cancer is squamous cell cancer.

In a particular embodiment, the cancer is endometrial cancer, more particularly endometroid cancer or endometrial serous cancer.

In a particular embodiment, the cancer is pancreatic cancer. More particularly, pancreatic cancer is exocrine pancreatic cancer (the most prevalent one) or neuroendocrine pancreatic cancer.

In a particular embodiment, the cancer is cholangiocarcinoma.

In a particular embodiment, the cancer is breast cancer, and more particularly resistant-recurrent breast cancer.

In a particular embodiment, the cancer is head and neck cancer.

In a particular embodiment, the cancer is a sarcoma. Sarcomas are cancers arising from connective tissue including muscles, bones, cartilage and fat. In particular embodiments, the sarcoma is e.g. osteosarcoma (of the bone), chondrosarcoma (of the cartilage), leiomyosarcoma (smooth muscles), rhabdomyosarcoma (skeletal muscles), mesothelial sarcoma or mesothelioma (membranous lining of body cavities), fibrosarcoma (fibrous tissue), angiosarcoma or hemangioendothelioma (blood vessels), liposarcoma (adipose or fatty tissue), glioma or astrocytoma (neurogenic connective tissue found in the brain), myxosarcoma (primitive embryonic connective tissue) or mesenchymous or mixed mesodermal tumor (mixed connective tissue types).

In a particular embodiment, the cancer is brain cancer. Particularly, the brain cancer is a glioma. More particularly, the glioma is a glioblastoma.

In a particular embodiment, the cancer is a germinoma. Germinomas refer to germ cell tumors, derived from pluripotent cells, most often presenting in the testicle or the ovary (seminoma and dysgerminoma, respectively).

In a particular embodiment, the cancer is a blastoma. Blastomas are cancers derived from immature precursor cells or embryonic tissue. Blastomas are more common in children than in older adults. In particular embodiments, the blastoma is e.g. hepatoblastoma, neuroblastoma, medulloblastoma, nephroblastoma, pancreatoblastoma, pleruropulmonary blastoma, retinoblastoma or glioblastoma multiforme. In a particular embodiment, the cancer is neuroblastoma.

Cancers that can be treated by the pharmaceutical combinations of the present invention are solid tumors, e.g. lung cancer, colorectal cancer, pancreatic cancer, larynx cancer, tongue cancer, breast cancer, ovarian cancer, prostate cancer, liver cancer, head and neck cancer, esophageal cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, dysgerminoma, embryonal carcinoma, Wilms' tumor, cervical cancer, testicular tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma.

In a particular embodiment, the cancer is metastatic or advanced cancer.

In a particular embodiment, the cancer is an haematological malignancy. The term "haematological malignancy" refers to a type of cancer that affects blood, bone marrow, and lymph nodes, and includes lymphomas, myelomas and leukemias. Historically, scientists and physicians have classified these diseases by their locations in the body, the appearance of affected cells under the microscope, and the natural progression of the diseases. In leukemia, the cancerous cells are discovered circulating in the blood and bone marrow, while in lymphoma, the cells tend to aggregate and form masses, or tumors, in lymphatic tissues. Myeloma is a tumor of the bone marrow, and involves a specific subset of white blood cells that produce a distinctive protein.

In particular embodiments, the haematological malignancy is a leukemia, a lymphoma or a myeloma.

In a particular embodiment, the haematological malignancy is leukemia. More particularly, the leukemia is e.g. acute myeloid leukaemia (AML), acute lymphoblastic leukaemia (ALL), chronic lymphocytic leukaemia (CLL), chronic myeloid leukaemia (CML), and acute monocytic leukemia (AMoL).

In a particular embodiment, the haematological malignancy is lymphoma. More particularly, the lymphoma is Hodgkin's lymphoma (HL) or Non-Hodgkin lymphoma (NHL).

In a particular embodiment, the haematological malignancy is myeloma, also known as multiple mieloma (i.e. cancer of plasma cells, normally white blood cells, that normally produce antibodies).

In another particular embodiment, the hematological malignancy is at precancerous stage. The term "precancerous stage", as used herein, refers to one hyperproliferative disorder or premalignancy condition that can develop into cancer.

### Administration of Compound (A) and/or Compound (B):

In relation to the herein discussed combination treatment is not essential if the two Compounds (A) and (B) are administrated e.g. simultaneous as a single composition or e.g. sequentially as two separate compositions. The important matter is that an effective amount of the compound/agent first administered is in the patient's body and/or has exerted its effect in the patient's body when the second compound/agent is administered.

When there herein is generically referred to Compound (B) it is understood to refer to any of Compound (B1), Compound (B2) and/or Compound (B3).

It may be preferred that the pharmaceutical combination as discussed herein is a single composition comprising both Compound (A) and Compound (B).

Compound (A) (in particular ABTL0812) is preferably administrated orally.

The administrated dose of Compound (A) (in particular ABTL0812) is preferably a daily dose of from 200 mg to 7000 mg, more preferably a daily dose of from 1500 mg to 5000 mg, even more preferably a daily dose of from 3000 mg to 4700 mg and most preferably a daily dose of from 3500 mg to 4300 mg.

Preferably, the daily dose of Compound (A) (in particular ABTL0812) is a daily dose administrated 3 times per day - most preferably as 3 times 1200-1400 mg.

Herein relevant human clinical studies with ABTL0812 have successfully been performed with a daily dose of 3900 mg - administrated as 3 times 1300 mg.

Accordingly, it is most preferred that Compound (A) is ABTL0812 and the administrated dose is from 3800 mg to 4000 mg (most preferably 3900 mg) - in particular when administrated as 3 times 1300 mg.

In relation to Compound (B), a preferred route of administration will generally depend on the Compound (B) interest.

The skilled person may routinely determine a preferred route of administration for a specific Compound (B) interest.

Preferred route of administration for preferred Compound (B) is briefly described below:
Temozolomide; - preferably administrated via oral capsules or tablets;
Topotecan; - preferably administrated intravenously via infusion solution;
Irinotecan; - preferably administrated intravenously via infusion solution;
Cyclophosphamide; - preferably administrated intravenously via infusion solution;
Fluorouracil; - preferably administrated intravenously via infusion solution;
Oxaliplatin; - preferably administrated intravenously via infusion solution;
Leucovorin; - preferably administrated intravenously via infusion solution;
Doxorubicin; - preferably administrated intravenously via infusion solution;
Bortezomib; - preferably administrated intravenously via infusion solution;
Olaparib; - preferably administrated via oral capsules or tablets;
Bevacizumab; - preferably administrated intravenously via infusion solution; Anti-PD1 checkpoint inhibitor antibody - preferably administrated intravenously via infusion solution.

### EXAMPLES

### EXAMPLE 1: ABTL0812 in combination with different/additional chemotherapeutic agents: In vitro assays

### 1.1: Cell viability assay of ABTL0812 alone or in combination with temozolomide in neuroblastoma

**Objectives:** To study the potential synergism of ABTL0812 when added to temozolomide in the neuroblastoma cell lines SK-N-BE(2) and LA1-5S. Temozolomide is a common chemotherapeutic agent used in e.g. second-line treatment backbones for high-risk neuroblastomas. Therefore, it is interesting to know whether there is any potentiation effect between ABTL0812 and temozolomide.

**Methods:** LA1-5S and SK-N-BE(2) cells were incubated with increasing concentrations of temozolomide (500 µM, 1000 µM and 1500 µM), and sublC50 fixed concentrations of ABTL0812. (30 µM for SK-N-BE(2) and 40 µM for LA1-5S). Cells were treated for 48 hours in IMDM with 0.5% FBS. Cell viability was evaluated by crystal violet assay. Different doses were assessed in six replicates and the results shown are the average of two independent experiments. Statistical analyses were performed according to the T-Test principle with GraphPad Prism^{®} 5.0 software.

**Results:** The addition of ABTL0812 at 30 µM or 40 µM increased temozolomide cytotoxicity. This increase was statistically significant at all concentrations (** p<0.01; *** p<0.001) - see Figure 1 herein.

**Conclusions:** ABTL0812 potentiates the cytotoxic effect of temozolomide *in vitro* in the neuroblastoma cell lines SK-N-BE(2) and LA1-5S. These results support an *in vivo* study of the combination of both drugs.

### 1.2: Cell viability assay of ABTL0812 alone or in combination with topotecan in neuroblastoma

**Objectives:** To study the potential synergism of ABTL0812 when added to topotecan in the neuroblastoma cell line LA1-5S. Topotecan is a common chemotherapeutic agent used in e.g. second-line treatment backbones for high-risk neuroblastomas. Therefore, it is interesting to know whether there is any potentiation effect between ABTL0812 and topotecan.

**Methods:** LA1-5S cells were incubated with increasing concentrations of topotecan (0.5 µM, 1 µM and 2 µM) and a fixed concentration of ABTL0812 (30 µM). Cells were treated for 72 hours in IMDM with 0.5% FBS. Cell viability was evaluated by crystal violet assay. Data is presented as mean ± SEM of three independent experiments. Statistical analyses were performed according to the T-Test principle with GraphPad Prism^{®} 5.0 software.

**Results:** The addition of ABTL0812 at 30 µM significantly increased topotecan cytotoxicity. (* p<0.05, ** p<0.01 compared to vehicle; ^{$} p<0.05 compared to the matching concentration of each drug as a single agent). See Figure 2 herein.

**Conclusions:** ABTL0812 potentiates the cytotoxic effect of topotecan *in vitro* in the neuroblastoma cell line LA1-5S. These results support an *in vivo* study of the combination of both drugs.

### 1.3: Cell viability assay of ABTL0812 alone or in combination with irinotecan in neuroblastoma

**Objectives:** To study the potential synergism of ABTL0812 when added to irinotecan in the neuroblastoma cell line LA1-5S. Irinotecan is a common chemotherapeutic agent used in e.g. second-line treatment backbones for high-risk neuroblastomas. Therefore, it is interesting to know whether there is any potentiation effect between ABTL0812 and irinotecan.

**Methods:** LA1-5S cells were incubated with increasing concentrations of irinotecan (4 µM, 8 µM and 16 µM) and a fixed concentration of ABTL0812 (30 µM). Cells were treated for 72 hours in IMDM with 0.5% FBS. Cell viability was evaluated by crystal violet assay. Data is presented as mean ± SEM of three independent experiments. Statistical analyses were performed according to the T-Test principle with GraphPad Prism^{®} 5.0 software (* p<0.05; ** p<0.01; *** p<0.001).

**Results:** The addition of ABTL0812 at 30 µM significantly increased irinotecan cytotoxicity. (* p<0.05, ** p<0.01 compared to vehicle; ^{#} p<0.05 compared to ABTL0812 as a single agent; ^{$} p<0.05 compared to the matching concentration of each drug as a single agent). See Figure 3 herein.

**Conclusions:** ABTL0812 potentiates the cytotoxic effect of irinotecan *in vitro* in the neuroblastoma cell line LA1-5S. These results support an *in vivo* study of the combination of both drugs.

### 1.4: Cell viability assay of ABTL0812 alone or in combination with cyclophosphamide in neuroblastoma

**Objectives:** To study the potential synergism of ABTL0812 when added to cyclophosphamide in the neuroblastoma cell line LA1-5S. Cyclophosphamide is a common chemotherapeutic agent used in e.g. second-line treatment backbones for high-risk neuroblastomas. Therefore, it is interesting to know whether there is any potentiation effect between ABTL0812 and cyclophosphamide.

**Methods:** LA1-5S cells were incubated with increasing concentrations of cyclophosphamide (1 µM, 1,5 µM and 2 µM) and a subIC50 fixed concentration of ABTL0812 (30 µM). Cells were treated for 72 hours in IMDM with 0.5% FBS. Cell viability was evaluated by crystal violet assay. Data is presented as mean ± SEM of three independent experiments. Statistical analyses were performed according to the T-Test principle with GraphPad Prism^{®} 5.0 software (* p<0.05; ** p<0.01; *** p<0.001).

**Results:** The addition of ABTL0812 at 30 µM significantly increased cyclophosphamide cytotoxicity at 1 µM. (^{$} p<0.05 compared to the matching concentration of each drug as a single agent). See Figure 4 herein.

**Conclusions:** ABTL0812 potentiates the cytotoxic effect of cyclophosphamide *in vitro* in the neuroblastoma cell line LA1-5S. These results support an *in vivo* study of the combination of both drugs.

### EXAMPLE 2: ABTL0812 in combination with targeted therapies: In vitro assays

### 2.1: Cell viability assay of ABTL0812 alone or in combination with Bortezomib in multiple myeloma

**Objectives:** To study the potential synergism of ABTL0812 when added to bortezomib in the multiple myeloma cell lines JJN-3 and OPM2. Bortezomib is a targeted therapy, a first in class pro-teasome inhibitor approved for treating multiple myeloma patients. Therefore, it is interesting to know whether there is any additive effect between both drugs.

**Methods:** JJN-3 and OPM2 cells were seeded in 24-well plates and treated with ABTL0812 (5 µM), bortezomib (0.5 and 1 nM respectively) or a combination of both drugs and left in the incubator for 48h (0.5% FBS). Cell viability was studied by the MTT assay and combination index was determined to evaluate a possible synergism.

**Results:** JJN-3 cells were incubated with ABTL0812 at 5 µM (Sub IC50 concentration) and with Bortezomib at 0.5 nM (Sub IC50 concentration), which induce around 10% and 15% of cell death respectively. When both drugs are combined, cell death is potentiated, inducing around 40% of cell death. Cell viability was evaluated in all cases by MTT assay and the Combination Index (CI), to evaluate synergism, was calculated according to the method of Chou and Talalay (Chou 2006; Chou 2010), as follows: CI = (D)1/(Dx)1 + (D)2/(Dx)2, where CI<1, =1, and >1 indicate synergism, additive effect, and antagonism, respectively. In the denominator, (Dx)1 is for D1"alone" that inhibits a system x%, and (Dx)2 is for D2"alone" that inhibits a system x%. In the numerators, (D)1 and (D)2"in combination" also inhibit x%. The CI for this combination is 0.049, which indicates a very high synergism. In the case of OPM2 cells, they were incubated with ABTL0812 at 5 µM (Sub IC50 concentration) and with Bortezomib at 1 nM (Sub IC50 concentration), which induce around 20% and 10% of cell death respectively. When both drugs are combined, cell death is potentiated, inducing around 55% of cell death. The combination index is 0.50, which indicates synergism - see Figure 5 herein.

Conclusions: ABTL0812 and bortezomib have strong synergistic effects *in vitro* in the multiple myeloma cell lines JJN-3 and OPM2. When both drugs are combined at sub IC50 concentrations, there is a potentiation of anticancer activity, thus demonstrating synergy between both drugs. These results open the opportunity for the *in vivo* combination of both drugs.

### EXAMPLE 3: ABTL0812 in combination with different chemotherapeutic agents: In vivo assays

### 3.1: Anticancer activity of ABTL0812 alone or in combination with FOLFIRINOX (5-FU, leucovorin, irinotecan and oxaliplatin) in a human pancreatic cancer xenograft model using MiaPAca2 cells implanted in nude mice

***Test System:*** Nu/nu female mice.

**Objective:** Investigate the anti-tumor activity of ABTL0812 alone and in combination with FOLFIRINOX, a standard of care therapy for treating pancreatic cancer.

**Methods:** Mice were injected with 5x10⁶ MiaPaca2 cells in one flank to induce tumor formation. When tumors had a volume of 100mm³ approximately, animals were homogenously randomized and the different treatments were started. ABTL0812 was administered by the oral route at 120 mg/kg/day. FOLFIRINOX chemotherapeutic combination was administered i.p. once a week for a total of four administrations. 5-FU at 30 mg/kg, leucovorin at 50 mg/kg, irinotecan at 50 mg/kg and oxaliplatin at 2.5 mg/kg were administered i.p. in two different days. 5-FU and leucovorin were administered on Tuesdays and irinotecan and oxaliplatin on Thursdays. Tumor volume and body weight were monitored 3 times a week.

**Results:** The combination of ABTL0812 with Folfirinox dramatically increased the therapeutic potential of chemotherapy and showed the highest tumor volume reduction compared with Folfirinox, ABTL0812 and vehicle groups. Statistical analysis showed that the combination therapy significantly improves the reduction of tumor growth compared to Folfirinox alone, which is the standard of care for treating advanced pancreatic cancer (p<0.001 by t-test). In addition, no decrease in body weight or hematological counts (not shown) were observed in any of the treatment groups, including those where ABTL0812 is administered with Folfirinox, suggesting this combination had no toxic effects. See Figure 6 herein.

**Conclusion:** ABTL0812 significantly potentiates Folfirinox anticancer effects without increasing toxicity in a human pancreatic cancer xenograft model using MiaPaca2 cells implanted in nude mice. Folfirinox is the standard of care for treating advanced pancreatic cancer patients, therefore these results suggest a combined therapy of ABTL0812 plus Folfirinox could have a clinical interest for the treatment of pancreatic cancer.

### 3.2: Anticancer activity of ABTL0812 alone or in combination with doxorubicin in a human endometrial cancer xenograft model using Ishikawa cells implanted in nude mice

**Objective:** Investigate the anti-tumor activity of ABTL0812 alone and in combination with doxorubicin, a reference second line treatment drug for the treatment of endometrial cancer.

**Methods:** Mice were injected with 4x10⁶ Ishikawa cells in one flank to induce tumor formation. When tumors had a volume of 50mm³ approximately, animals were homogenously randomized and the different treatments were started. ABTL0812 was administered by the oral route at 120 mg/kg/day. Doxorubicin 5mg/kg was administered intra-peritoneally once a week. Tumor volume and body weight were monitored 3 times a week.

**Results:** ABTL0812 and doxorubicin significantly reduced tumor volume when compared to control animals (ANOVA followed by t-test). ABTL0812 efficacy was indeed similarly to the efficacy observed for doxorubicin treatment. Interestingly, ABTL0812 potentiated the antitumor effect of docetaxel. Statistical analysis showed that this combination therapy significantly improves the reduction of tumor growth compared to doxorubicin alone (*p<0.05 by t-test). In addition, no decrease in body weight or hematological counts (not shown) were observed in any of the treatment groups, including those where ABTL0812 is administered with doxorubicin, suggesting this combination had no toxic effects. See Figure 7 herein.

**Conclusion:** ABTL0812 reduces tumor growth in xenograft models of endometrial cancer derived from Ishikawa cells. In this model, ABTL0812 has an efficacy that is similar to the SOC doxorubicin. ABTL0812 potentiates the antitumor activity of doxorubicin with no toxic effect. These results suggest a combined therapy of ABTL0812 plus Doxorubicin could have a clinical interest for the treatment of endometrial cancer.

### 3.3: Anticancer activity of ABTL0812 alone or in combination with temozolomide in a human glioblastoma orthotopic xenograft model using U87MG cells implanted in the brains of nude mice

**Objective:** To investigate the anti-tumor activity of ABTL0812 alone and in combination with temozolomide, a reference drug for the treatment of glioblastoma.

**Methods:** Mice were intra-cerebrally injected with luciferase transfected U87MG cells. Animals were randomized 5 days after cell inoculation and treatments started. ABTL0812 was administered orally at 240 mg/kg 5 days/week; and temozolomide orally at 32 mg/Kg the first 5 days. Tumors were measured by quantification of bioluminescence intensity (BLI) of the region of interest.

**Results:** ABTL0812 and temozolomide as single agents significantly increased the disease-free survival of animals bearing glioblastoma tumors in the brain. Interestingly, the combination of ABTL0812 with temozolomide was significantly more efficacious than the single treatments. See Figure 8 herein.

**Conclusion:** ABTL0812 as single agent increases the disease-free survival of mice bearing tumors and potentiates the antitumor activity of temozolomide. These results suggest that the combination therapy of ABTL0812 plus temozolomide might have a clinical interest for the treatment of glioblastoma.

### EXAMPLE 4: ABTL0812 in combination with radiotherapy: In vivo assays

### 4.1: Anticancer activity of ABTL0812 alone or in combination with radiotherapy in a human glioblastoma subcutaneous xenograft model using U87MG and T98G cells implanted in nude mice

**Objective:** To investigate the anti-tumor activity of ABTL0812 alone and in combination with radiotherapy, a main therapy strategy for glioblastoma.

**Methods:** Mice were injected subcutaneously with 1x10⁶ U87MG or T98G cells in each flank to induce tumor formation. When tumors had a volume of 0.8-1.3 cm³, animals were homogenously randomized and the different treatments were started. ABTL0812 was administered orally at 240mg/kg 5 days/week and radiotherapy as single dose of 4 Gy administered at day 3.

**Results:** ABTL0812 as a single agent significantly decreased the growth of glioblastoma subcutaneous tumors. Moreover, the combination of ABTL0812 with radiotherapy was significantly more efficacious than ABTL0812 or radiotherapy as single treatments. See Figure 9 herein.

**Conclusion:** ABTL0812 as single agent decreases the growth of glioblastoma tumors and potentiates the antitumor activity of radiotherapy. These results suggest that the combination therapy of ABTL0812 plus radiotherapy might have a clinical interest for the treatment of glioblastoma.

### 4.2: Anticancer activity of ABTL0812 alone or in combination with radiotherapy in a human glioblastoma orthotopic xenograft model using U87MG cells implanted in the brains of nude mice

**Objective:** To investigate the anti-tumor activity of ABTL0812 alone and in combination with radiotherapy, a main therapy strategy for glioblastoma.

**Methods:** Mice were intra-cerebrally injected with luciferase transfected U87MG cells. Animals were randomized 5 days after cell inoculation and treatments started. ABTL0812 was administered orally at 240 mg/kg 5 days/week and radiotherapy as single dose of 4 Gy administered at day 10. Tumors were measured by quantification of bioluminescence intensity (BLI) of the region of interest.

**Results:** ABTL0812 and radiotherapy as single agents significantly increased the disease-free survival of animals bearing glioblastoma tumors in the brain. Interestingly, the combination of ABTL0812 with radiotherapy was significantly more efficacious than the single treatments. See Figure 10 herein.

**Conclusion:** ABTL0812 as single agent increases the disease-free survival of mice bearing tumors and potentiates the antitumor activity of radiotherapy. These results suggest that the combination therapy of ABTL0812 plus radiotherapy might have a clinical interest for the treatment of glioblastoma.

### EXAMPLE 5: ABTL0812 in combination with targeted therapies: In vivo assays

### 5.1: Anticancer activity of ABTL0812 alone or in combination with olaparib in a human endometrial cancer model using Ishikawa cells implanted in nude mice

**Objective:** Investigate the anti-tumor activity of ABTL0812 alone and in combination with olaparib, a reference drug for the treatment of endometrial cancer.

**Methods:** Mice were injected with 4x10⁶ Ishikawa cells in one flank to induce tumor formation. When tumors had a volume of 50mm³ approximately, animals were homogenously randomized and the different treatments were started. ABTL0812 was administered by the oral route at 120 mg/kg/day. Olaparib was administered by the oral route 50 mg/kg/day. Tumor volume and body weight were monitored 3 times a week.

**Results:** ABTL0812 and olaparib significantly reduced tumor volume when compared to control animals (ANOVA followed by t-test). ABTL0812 efficacy was indeed similarly to the efficacy observed for olaparib treatment. Interestingly, ABTL0812 potentiated the antitumor effect of docetaxel. Statistical analysis showed that this combination therapy significantly improves the reduction of tumor growth compared to olaparib alone (**p<0.01 by t-test). In addition, no decrease in body weight or hematological counts (not shown) were observed in any of the treatment groups, including those where ABTL0812 is administered with olaparib, suggesting this combination had no toxic effects. See Figure 11 herein.

**Conclusion:** ABTL0812 reduces tumor growth in xenograft models of endometrial cancer derived from Ishikawa cells. In this model, ABTL0812 has an efficacy that is similar to olaparib treatment. ABTL0812 potentiates the antitumor activity of olaparib with no toxic effect. These results suggest a combined therapy of ABTL0812 plus Olaparib could have a clinical interest for the treatment of endometrial cancer.

### 5.2: Anticancer activity of ABTL0812 alone or in combination with bevacizumab in a human endometrial cancer model using Ishikawa cells implanted in nude mice

**Objective:** Investigate the anti-tumor activity of ABTL0812 alone and in combination with bevacizumab, that has shown potential efficacy in endometrial cancer.

**Methods:** Mice were injected with 4x10⁶ Ishikawa cells in one flank to induce tumor formation. When tumors had a volume of 50mm³ approximately, animals were homogenously randomized and the different treatments were started. ABTL0812 was administered by the oral route at 120 mg/kg/day. Bevacizumab was administered ip at 100 µg/dose, every four days up to four administration. Tumor volume and body weight were monitored 3 times a week.

**Results:** ABTL0812 and bevacizumab significantly reduced tumor volume when compared to control animals (ANOVA followed by t-test). ABTL0812 efficacy was indeed similarly to the efficacy observed for bevacizumab treatment. Interestingly, ABTL0812 potentiated the antitumor effect of docetaxel. Statistical analysis showed that this combination therapy significantly improves the reduction of tumor growth compared to bevacizumab alone (*p<0.05 by t-test). In addition, no decrease in body weight or hematological counts (not shown) were observed in any of the treatment groups, including those where ABTL0812 is administered with bevacizumab, suggesting this combination had no toxic effects. See Figure 12 herein.

Conclusion: ABTL0812 reduces tumor growth in xenograft models of endometrial cancer derived from Ishikawa cells. In this model, ABTL0812 has an efficacy that is similar to bevacizumab treatment. ABTL0812 potentiates the antitumor activity of bevacizumab with no toxic effect. These results suggest a combined therapy of ABTL0812 plus Bevacizumab could have a clinical interest for the treatment of endometrial cancer.

### EXAMPLE 6: Immunomodulatory effects of ABTL0812: In vitro assays

### 6.1: Immunomodulatory effects of ABTL0812 on human THP-1 human macrophage cells by potentiation of M1 pro-inflammatory and suppression of M2 anti-inflammatory phenotypes

**Objective:** Investigate the immunomodulatory effect of ABTL0812 on the polarization of macrophages to a M1 phenotype (pro-inflammatory and anti-tumoral) and to a M2 phenotype (anti-inflammatory pro-tumoral), which would influence the tumor microenvironment upon ABTL0812 treatment.

**Methods:** THP-1 monocytes growing in suspension are differentiated to macrophages by incubation with PMA for 24 hours, inducing their attachment to the plates. Once THP-1 are differentiated to macrophages, these are polarized to M1 by incubation with LPS for 6 or 24 hours in the presence of ABTL0812 (50 or 100 µM). In parallel, differentiated macrophages are polarized to M2 by incubation with IL-4 and IL-13 for 24 hours in the presence of ABTL0812 (50 µM). Then, polarized macrophages are lysed, total RNA extracted, retrotranscribed to cDNA and mRNA levels of IL1β, TNFα (M1 markers) and IL-10 (M2 marker) were evaluated by RT-qPCR using specific probes.

**Results:** ABTL0812 immunomodulatory effects significantly potentiates IL-1β and TNFα mRNA levels when macrophages are polarized to M1, and significantly suppresses IL-10 mRNA levels when macrophages are polarized to M2. (t-test **p<0.01 and ***p<0.001). When differentiated macrophages are incubated with ABTL0812 alone without polarizing them, ABTL0812 is able to significantly induce the expression of IL-1β, highlighting its immunomodulatory effects on human THP-1 cells. See Figure 13 herein.

**Conclusion:** ABTL0812 potentiates the polarization of human THP-1 monocytes to M1, significantly increasing the gene expression of IL-1β and TNFα, which promotes a pro-inflammatory environment that would exert anti-tumor effects. Moreover, ABTL0812 suppresses the polarization of human THP-1 monocytes to M2, significantly decreasing the gene expression of IL-10, avoiding the immunosuppression mediated by M2 macrophages, a common mechanism used by tumor cells to evade immune system. These results suggest that ABTL0812, apart from its anti-cancer effect on tumor cells, stimulates immune system to a pro-inflammatory phenotype, recruiting other immune cells as cytotoxic T lymphocytes, thus making a "cold" tumor which induces immune system suppression, to a "hot" and immunogenic tumor, highlighting the potential combination of ABTL0812 with in particular immune checkpoint inhibitors to potentiate anticancer efficacy by promoting a pro-inflammatory and anti-tumor microenvironment.

### 6.2: Induction of PDL1 expression in cancer cells treated with ABTL0812

**Objective:** Investigate the immunomodulatory effect of ABTL0812 on the expression of PDL1 in cancer cells, alone or in combination with IFNγ, a well described master regulator of PDL1 expression.

**Methods:** Human cancer cell lines were incubated with 100 µM of ABTL0812 during 48 hours and collected for staining with anti-PDL1 antibody labeled with a fluorophore. After staining of cells with anti-PDL1 antibody, cells were run on Flow Cytometry (Facs Canto) to analyzed PDL1 levels in cancer cells. Additionally, we incubated Panc-1 cells with ABTL0812 (50 µM), IFNy (2.5 ng/ml), a master regulator of PDL1 expression or a combination with both. Then, cells were collected, stained with anti-PDL1 antibody and analyzed by Flow Cytometry for the expression of PDL1.

**Results:** ABTL0812 induces the expression of PDL1 in all cancer cell lines tested with similar potency among them, ranging from 34% (MiaPaca2 cells) to 86% increase (Capan-2 cells) from basal levels (T test *p<0.05, **p<0.01). When PDL1 levels were analyzed in Panc-1 cells incubated with IFNy with or without ABTL0812, IFNy induces significantly higher levels of PDL1 compared with ABTL0812 (T test **p<0.01). Interestingly, cells incubated with ABTL0812 and IFNy induced PDL1 levels similar to the sum of ABTL0812-mediated effect and IFNy-mediated effect. ABTL0812 in combination with IFNy induces significantly higher PDL1 levels compared with ABTL0812 treatment (T test ***p<0.001), although this increase is not significant compared with IFNy treatment, suggesting an additive effect on PDL1 expression when both drugs are administered together. See Figure 14 herein.

**Conclusion:** ABTL0812 induces PDL1 expression in human pancreatic and endometrial cancer cells. IFNy, a master regulator of PDL1 expression, induces higher PDL1 levels compared with ABTL0812, although when both drugs are administered together, there is an additive effect on PDL1 expression levels, inducing higher levels. ABTL0812 is cytotoxic against cancer cells, and stimulates macrophages towards a pro-inflammatory phenotype, which among others, produces high levels of IFNy. These results highlight the potential combination of ABTL0812 with immune checkpoint inhibitors, since the induction of PDL1 levels mediated will make cancer cells targetable for immune checkpoint inhibitors.

### 6.3: Induction of pro-inflammatory environment in macrophages

**Objective:** After evaluating the immunomodulatory effects of ABTL0812 on human macrophages, the effect of ABTL0812 on cancer cells and how conditioned media of this ABTL0812-treated cancer cells affect viability and polarization of human macrophages were investigated.

**Methods:** MiaPaca2 cells were incubated with 40 µM of ABTL0812 during 72 hours and conditioned media (RPMI) of these ABTL0812-treated cells were collected. In parallel, human THP-1 cells were differentiated to macrophages by incubation with PMA (Phorbol myristate acetate) for 24 hours, inducing their attachment to the plates. Then, conditioned media from ABTL0812-treated MiaPaca2 cells was transferred to PMA-activated THP-1 macrophages and incubated during 24 hours for RT-qPCR analysis of M1 phenotype markers IL-1β and TNF-α, or during 48 hours for cell viability studies using the MTT assay. For M1 marker analysis, cells were collected, RNA extracted, retrotranscribed to cDNA and mRNA levels of IL-1β and TNF-α assessed by RT-qPCR.

**Results:** Previous results have shown that ABTL0812 is cytotoxic against MiaPaca2 cells, with an IC50 of 50µM, which also induces the release of different factors to the culture media. When this media of ABTL0812-treated MiaPaca2 cells is transferred to activated THP-1 macrophages, this induces their metabolic activation, significantly increasing THP-1 cell viability. Moreover, ABTL0812-conditioned media induces the polarization of THP-1 macrophages towards a pro-inflammatory anti-tumoral phenotype, significantly increasing the gene expression of IL-1β and TNF-α, thus confirming the immunomodulatory effects of ABTL0812 mediated by its effect on cancer cells. See Figure 15 herein.

**Conclusion:** ABTL0812 shows immunomodulatory effects not only through direct effect on macrophage polarization, but also through its effect on cancer cells, since ABTL0812-conditioned media from MiaPaca2 cells is able not only to increase viability and metabolic activity on human THP-1 macrophages, but also inducing their polarization to M1 by increasing the expression of IL-1β and TNF-α in these macrophages. These data, in combination with the induction of ICD (Example 6.7) and the inhibition of secretion of immunosuppressive factors (Example 6.6), strongly suggest that ABTL0812 is able to immunomodulate the tumor microenvironment by its anticancer effect on cancer cells, which in turn converts "cold" tumors into "hot" tumors and targetable for immune system, in addition to its direct effect on human macrophages, therefore highlighting its potential combination with immune checkpoint inhibitors to potentiate the anticancer efficacy.

### 6.4: Induction of PDL1 expression in cancer cells treated with ABTL0812

**Objective:** Investigate the immunomodulatory effect of ABTL0812 on the expression of PDL1 in cancer cells, potentially exposing cancer cells to immunotherapy with immune checkpoint inhibitors.

**Methods:** Human pancreatic cancer cell lines (MiaPaca2, Panc-1, Capan-2 and SU.86.86) and human endometrial cancer cell lines (Ishikawa, ANC3, Hec-1A, Ark1 and Ark2), were incubated with ABTL0812 at doses ranging from 0 to 80 µM for 24 hours. Afterwards cells were collected and stained with anti-PDL1 antibody for flow cytometry analysis of percentage of PDL1 positive cells.

**Results:** ABTL0812 induces the expression of PDL1 in all cancer cell lines tested ranging from 34% to 86% increase in pancreatic cancer cells, and from 10 to 35% in endometrial cancer cells (T test *p<0.05, **p<0.01). See Figure 19 herein.

**Conclusion:** ABTL0812 induces PDL1 expression in human pancreatic and endometrial cancer cells, potentially making cancer cells targetable by immune checkpoint inhibitors. These results support the potential synergy of ABTL0812 and immunotherapy for treating cancer.

### 6.5: Inhibition of PD1 expression in primary human CD4 and CD8 T cells from peripheral blood of healthy donors treated with ABTL0812 ex vitro

**Objective:** Investigate the immunomodulatory effect of ABTL0812 on the expression of PD1 in primary human T cells activated or not with CD3 and CD28.

**Methods:** Human PBMCs were purified from peripheral blood of healthy donors using Ficoll and cultured *in vitro.* Human T cells were activated by incubation with IL-2 and CD3 and CD28 antibodies for 10 days and then treated with ABTL0812 for 6 hours. Finally, levels of PD1 expressed in the membrane of T cells was analyzed by flow cytometry using specific antibodies. Results are indicated as % of positive cells and show the average of three independent experiments (t-test **p<0.01 and ***p<0.001).

**Results:** ABTL0812 induces the inhibition of PD1 expression in non-activated and in activated human primary CD4 and CD8 T lymphocytes. See Figure 20 herein.

**Conclusion:** ABTL0812 induces the inhibition of PD1 expression in both, activated and non-activated primary human CD4 and CD8 T cells purified from the blood of healthy donors. This could potentially help to boost the immune system against cancer cells, through blocking the immunosuppression and T cell inactivation mediated by PD1.

### 6.6: Immunomodulatory effects of ABTL0812 on the secretome of human pancreatic cancer cells

**Objective:** Investigate the immunomodulatory effect of ABTL0812 on the secretome of cancer cells by analyzing the secreted factors in the culture media with a protein microarray that detects up to 38 different chemokines.

**Methods:** Human pancreatic cancer cells were treated with ABTL0812 at 100 uM for 24 hours and culture media was collected for its incubation using the RayBio C-Series Human Chemokine Antibody Array C1 (RayBiotec). Culture media was incubated with a membrane containing antibodies against 38 different chemokines. Afterwards, the membrane was incubated with secondary antibodies and further developed using HRP substrate. The intensity of the signal was assessed using densitometry and graphic shows results of three different biological replicates.

**Results:** ABTL0812 immunomodulatory effects induces a decrease in immunosuppressive chemokines CXCL6 (related to immunosuppression, invasion and bad prognosis); CXCL16 (promotes tumor invasion and it is upregulated in pancreatic cancer); angiogenin (promotes immunosuppression and angiogenesis); and CCL5 (promotes Tregs tumor infiltration and immunosuppression). See Figure 21 herein.

**Conclusion:** ABTL0812 promotes the inhibition of the release of immunosuppressive factors in human pancreatic cancer cells. These data suggest that ABTL0812 effect on cancer cells secretome modulates tumor microenvironment towards a more pro-inflammatory and anti-tumor phenotypes, inhibiting the secretion of immunosuppressor factors.

### 6.7: Immunomodulatory effects of ABTL0812 on human pancreatic cancer cells by the induction of immunogenic cell death

**Objective:** Investigate the immunomodulatory effect of ABTL0812 on cancer cells by the evaluation of Immunogenic Cell Death (ICD) induction.

**Methods:** Human pancreatic cancer cells were treated with increasing concentrations of ABTL0812 (ranging from 0 to 150 µM) for 24 hours, and ICD hallmarks extracellular Hmgb1 and ATP, surface Calreticulin, and caspases 3 and 8 activation, were evaluated by ELISA (Hmgb1 and ATP), flow cytometry (Calreticulin) and immunoblotting (Caspases 3 and 8). For extracellular Hmgb1 and ATP, culture media from ABTL0812 treated cells were collected and incubated with specific antibodies for further detection using colorimetric assay. For surface Calreticulin, cancer cells were collected and incubated with specific antibody for further detection using flow cytometry. For Caspase 3 and 8 activation, cancer cells were collected, protein lysate obtained, incubated with specific antibodies for further detection using immunoblotting and finally quantified using densitometry.

**Results:** ABTL0812 immunomodulatory effects induces a dose dependent increase in all ICD hallmarks: extracellular Hmgb1 and ATP, surface Calreticulin and Caspase 3 and 8 activation, as detected by ELISA, luciferase assay, flow cytometry and fluorescence-based substrate assays, respectively (t-test **p<0.01 and ***p<0.001). See Figure 22 herein. Similar results were obtained with different human pancreatic cancer cell lines; in Figure 23 the results in MiaPaca2 cells are shown as a representative experiment.

**Conclusion:** ABTL0812 induces ICD in human pancreatic cancer cells, as indicated by a dose-dependent increase of ICD markers: extracellular Hmgb1 and ATP, surface Calreticulin, and caspases 3 and 8 activation. These results suggest that ABTL0812 could induce ICD in tumors, making them more immunogenic and targetable for the immune system, helping make a "cold" tumor which induces immune system suppression, to a "hot" and immunogenic tumor. These data support the potential combination of ABTL0812 with immunotherapy to potentiate anticancer efficacy.

### 6.8: Immunomodulatory effects of ABTL0812 on human immortalized THP-1 and human primary macrophage cells by potentiation of M1 pro-inflammatory and suppression of M2 anti-inflammatory phenotypes

**Objective:** To investigate the immunomodulatory effect of ABTL0812 on the polarization of immortalized and primary macrophages to a M1 phenotype (pro-inflammatory and anti-tumoral) and to a M2 phenotype (anti-inflammatory pro-tumoral), which would influence the tumor microenvironment upon ABTL0812 treatment.

**Methods:** THP-1 monocytes growing in suspension were differentiated to macrophages by incubation with PMA for 24 hours, inducing their attachment to the plates. Monocytes were differentiated to macrophages incubating with M-CSF1 at 20 ng/mL for 7 days. In parallel, whole blood was collected from healthy donors, and circulating monocytes were purified using immunomagnetic separation. Monocytes were selected using anti-CD14 antibodies bound to magnetic beads, which were retained in the magnetic column and further eluted for culturing *in vitro.* After obtaining activated macrophages (immortalized and primary), these were polarized to M1 by incubation with LPS + IFNy for 6 hours in the presence of ABTL0812 at 100 µM. In parallel, differentiated macrophages were polarized to M2 by incubation with IL-4 and IL-13 for 24 hours in the presence of ABTL0812 (100 µM). Afterwards, polarized macrophages were lysed, total RNA extracted, retrotranscribed to cDNA and mRNA levels of IL1β, TNFα (M1 markers) and IL-10 (M2 marker) were evaluated by RT-qPCR using specific probes.

**Results:** ABTL0812 immunomodulatory effects significantly potentiates IL-1β and TNFα mRNA levels when macrophages are polarized to M1, and significantly suppresses IL-10 mRNA levels when macrophages are polarized to M2 (t-test **p<0.01 and ***p<0.001). When differentiated macrophages are incubated with ABTL0812 alone without polarizing them, ABTL0812 significantly induces the expression of IL-1β, highlighting its immunomodulatory effects on human THP-1 cells. See Figure 24 herein.

Conclusion: ABTL0812 potentiates the polarization of both human THP-1 and human primary macrophages to M1, significantly increasing the gene expression of IL-1β and TNFα, which promotes a pro-inflammatory environment that would exert anti-tumor effects. Moreover, ABTL0812 suppresses the polarization of human THP-1 monocytes to M2, significantly decreasing the gene expression of IL-10, avoiding the immunosuppression mediated by M2 macrophages, a common mechanism used by tumor cells to evade immune system. These results suggest that ABTL0812, apart from its anticancer effect on tumor cells, stimulates immune system to a pro-inflammatory phenotype, recruiting other immune cells as cytotoxic T lymphocytes, thus making a "cold" tumor, which induces immune system suppression, to a "hot" and immunogenic tumor. These results support the potential combination of ABTL0812 with immunotherapy to potentiate anticancer efficacy by promoting a pro-inflammatory and anti-tumor microenvironment.

### 6.9: Immunomodulatory effects of ABTL0812 on the secretome of human immortalized THP1 macrophages and human primary macrophages by protein microarray

**Objective:** Investigate the immunomodulatory effect of ABTL0812 on the secretome of immortalized and primary macrophages, analyzing the secreted factors in the culture media with a protein microarray that detects up to 42 different cytokines after treatment with ABTL0812.

**Methods:** Whole blood was collected from healthy donors, and circulating monocytes were purified using immunomagnetic separation. Monocytes were selected using anti-CD14 antibodies bound to magnetic beads, which were retained in the magnetic column and further eluted for culturing *in vitro.* Monocytes were differentiated to macrophages incubating with M-CSF1 at 20 ng/mL for 7 days. Once primary monocytes were differentiated to macrophages, these were polarized to M1 by incubation with LPS for 6 or 24 hours in the presence of ABTL0812 (50 or 100 µM). In parallel, differentiated macrophages were polarized to M2 by incubation with IL-4 and IL-13 for 24 hours in the presence of ABTL0812 (50 µM). THP-1 monocytes growing in suspension were differentiated to macrophages by incubation with PMA for 24 hours, inducing their attachment to the plates. Once THP-1 were differentiated to macrophages, these were polarized to M1 by incubation with LPS for 6 or 24 hours in the presence of ABTL0812 (50 µM). In parallel, differentiated macrophages were polarized to M2 by incubation with IL-4 and IL-13 for 24 hours in the presence of ABTL0812 (50 µM). The culture media from ABTL0812 treated M1, M2 and differentiated macrophages (M0) were incubated using the RayBio C-Series Human Cytokine Antibody Array C3 (RayBiotec). Culture media was incubated with a PVDF (Polyvinylidene fluoride) membrane containing antibodies against 42 different cytokines. After incubation of culture media, the membrane was incubated with secondary antibodies and further developed using HRP (Horseradish peroxidase) substrate. The intensity of the signal was assessed using densitometry and graphic shows results of three different biological replicates.

**Results:** ABTL0812 immunomodulatory effects induce a decrease in immunosuppressive chemokines and upregulation of different pro-inflammatory factors such as IL-1β and TNF-α. Among all immunosuppressive cytokines that are inhibited upon ABTL0812 treatment, five of them were common in immortalized THP1 cells and in primary macrophages: IL-10 (associated to immunosuppression, invasion and poor prognosis in different cancers); CCL22 (associated to immunosuppression, invasion and poor prognosis in different cancers); CCL17 (associated to immunosuppression, invasion and poor prognosis in different cancers); CCL8 (associated to immunosuppression, proliferation and invasion); and CCL7 (associated to immunosuppression and invasion). See Figure 25 herein.

**Conclusion:** ABTL0812 promotes the secretion of pro-inflammatory factors and suppresses the release of immunosuppressive factors in cancer cells. These data, in combination with the potentiation of M1 macrophage phenotype and the suppression of M2 phenotypes (Example 6.8), suggest that ABTL0812 can promote a pro-inflammatory anti-tumor environment towards its action on the immune cells. These results support the potential combination of ABTL0812 with immunotherapy to increase the therapeutic effect against tumors, particularly, in those highly immunosuppressive, such as pancreatic cancers.

### 6.10: ABTL0812 immunomodulatory effects increases T cells cytotoxicity in cancer cells

**Objective:** Investigate the immunomodulatory effect of ABTL0812 on the cancer cells co-cultures with activated T cells.

**Methods:** human PBMCs were purified from peripheral blood of healthy donors using Ficoll and cultured *in vitro.* Human T cells were activated by incubation with IL-2 and CD3 and CD28 antibodies for 10 days. In parallel, Ishikawa endometrial cancer cells were treated with ABTL0812 at 50 µM for 6 hours and then co-cultured with activated T lymphocytes for 24 hours. Then, cell viability was assessed by the MTT assay. Untreated Ishikawa cells were used as controls. Results show average of three different experiments (t-test *p<0.05).

**Results:** ABTL0812 effect on cancer cells potentiates the cytotoxic effect of activated T cells, compared with cancer cells non-treated with ABTL0812, which correlates with the promotion of pro-inflammatory environment mediated by ABTL0812 on cancer cells. See Figure 26 herein.

**Conclusion:** ABTL0812 effect on cancer cells promotes the activation of the immune system, potentiating the cytotoxic effect of activated primary cells against cancer cells.

### EXAMPLE 7: Immunomodulatory effects of ABTL0812: In vivo assays

### 7.1: Induction of PDL1 expression in cancer cells treated with ABTL0812 in a human lung cancer xenograft model implanted with H157 cells and in a human pancreatic cancer model implanted with MiaPaca2 cells

**Objective:** Validate the induction of PDL1 expression by ABTL0812 observed *in vitro,* in two *in vivo* model using human squamous NSLC cell line H157 and human pancreatic cancer cell line MiaPAca2 implanted in nude mice.

**Methods:** Mice were injected with 4x10⁶ H157 cells or 5x10⁶ MiaPAca2 cells in one flank to induce tumor formation. When tumors had a volume of 50mm³ approximately, animals were homogenously randomized and the different treatments were started. ABTL0812 was administered by the oral route at 120 mg/kg/day for three weeks. After treatment, animals were sacrificed, tumor extracted and protein levels of PDL1 in cancer cells analyzed by western blot.

**Results:** ABTL0812 Increases protein levels of PDL1 in tumor *in vivo,* further validating previous *in vitro* results (Figure 14). These results highlight the potential combination of ABTL0812 with immune checkpoint inhibitors, since the induction of PDL1 levels mediated will make cancer cells targetable for immune checkpoint inhibitors.

### 7.2: Induction of T cell infiltration within tumor lesions in uterus from female mice with endometrial carcinoma treated with ABTL0812

**Objective:** Validate the anticancer efficacy and the immunomodulatory effects of ABTL0812 in a syngeneic model of endometrial carcinogenesis induced by the deletion of PTEN in epithelial cells, which leads to the development of hyperplasias that finally lead to endometrial intraepithelial neoplasias.

**Methods:** Mice were injected with tamoxifen to induce PTEN deletion. Three weeks after tamoxifen administration, when animal has developed hyperplasias, animals were administered with ABTL0812 at 120 mg/kg daily or with vehicle during 3 weeks. At that time, vehicle treated animals (6 weeks after tamoxifen injection) develop neoplasias, which are quantified by immunohistochem-istry of extracted uterus. After ABTL0812 treatment, animals were sacrificed, uterus extracted in paraffin-embedded for further immunohistochemical analysis by hematoxylin-eosin staining for carcinogenesis evaluation or with anti-CD3 to evaluate T lymphocyte infiltration within tumoral lesions, indicative of tumor microenvironment immunomodulation.

**Results:** Mice undergoing endometrial carcinogenesis treated with ABTL0812 show a significant reduction in the development of endometrial intraepithelial neoplasias (EIN), stopping carcinogenesis progression in hyperplasias, where vehicle treated mice showed 80% of animals with EIN compared with 80% of ABTL0812-treated animals with hyperplasias, analyzed by hematoxylin-eosin staining. When treated uterus were analyzed for the expression of CD3 T lymphocytes, ABTL0812 induce the infiltration of CD3 T lymphocytes within tumoral lesions, while vehicle treated animals showed CD3 T lymphocytes in the surrounding stroma lining the tumoral lesions, without infiltrating within tumors. See Figure 23 herein.

**Conclusion:** ABTL0812 immunomodulatory effects *in vivo* show how it induces the infiltration of T lymphocytes within tumoral lesions, indicative of the presence of a pro-inflammatory anti-tumoral microenvironment that favors the infiltration of immune cells to kill cancer cells. These data correlate with the good efficacy results, where ABTL0812 is able to stop endometrial carcinogenesis progression in hyperplasias, while vehicle treated animals show intraepithelial neoplasias, highlighting its potential combination with immune checkpoint inhibitors to potentiate anticancer efficacy.

### EXAMPLE 8: ABTL0812 in combination with immunotherapeutic agents: In vivo assays

### 8.1: Anticancer activity of ABTL0812 alone or in combination with anti-PD1 in a murine model of lung cancer using LLC1 cells implanted in C57BL6 mice

**Objective:** Investigate the anti-tumor activity of ABTL0812 alone and in combination with anti-PD1 antibody, a reference drug for the treatment of lung cancer, in terms of survival and based on end point criteria focused on tumors higher than 800 mm³ or the identification of clinical signs of toxicity or suffering. Pembrolizumab is an anti-PD1 checkpoint inhibitor for human use - in this Example was used a corresponding modified version optimized for use in the murine model used in this Example.

**Methods:** Lewis Lung Carcinoma cells (LLC1) are a highly tumorigenic murine cell line initially derived from the lung of a C57BL mouse implanted with a primary Lewis Lung carcinoma. These cells can be subcutaneously grown in syngeneic C57BL6 mice, where they develop a very aggressive tumor and can be used to evaluate immunotherapy treatments. C57BL6 mice were injected with 0.25x10⁶ LLC1 cells in one flank to induce tumor formation. When tumors had a volume of 50mm³ approximately, animals were homogenously randomized into treatment groups (n=7), and the different treatments were started. ABTL0812 was administered by the oral route at 120 mg/kg/day. Anti-PD1 antibody was administered i.p. at 100 ug/dose every three days for up a total of four administrations. Tumor volume and body weight were monitored 3 times a week. End point criteria was based on tumors over 800 mm3, or clinical signs of toxicity, distress or suffering indicative of animal euthanasia.

**Results:** ABTL0812 administered alone is able to slightly increase mice survival compared with vehicle and anti-PD1 treatment groups, showing 15% of survival after 14 days of treatment, compared with 0% survival in vehicle and anti-PD1 groups. Interestingly, the double combination ABTL0812 + anti-PD1 antibody shows the best survival, with 38% survival after 14 days of treatment. After 9 days of treatment, vehicle group shows 29% of survival, anti-PD1 group 15% of survival, ABTL0812 group 43% of survival and ABTL0812 + anti-PD1 treatment 62% of survival. See Figure 16 herein.

**Conclusion:** ABTL0812 in combination with anti-PD1 treatment (immune checkpoint inhibitor), significantly increases mice survival compared with vehicle, anti-PD1 and ABTL0812 treatments. ABTL0812 administered alone slightly increased mice survival after 14 days of treatment, although its effect administered alone is higher at shorter times. These data suggest a synergistic effect between ABTL0812 and anti-PD1 treatments, leading to an increase in mice survival and highlighting its potential combination for human patients.

### 8.2: Anticancer activity of ABTL0812 alone or in combination with anti-PD1/paclitaxel/carboplatin in a murine model of lung cancer using LLC1 cells implanted in C57BL6 mice

**Objective:** Investigate the anti-tumor activity of ABTL0812 alone and in combination with anti-PD1 antibody and carboplatin/paclitaxel, a combinatory reference therapy for the treatment of human lung cancer, evaluating potential synergism between ABTL0812 and anti-PD1+carboplatin/paclitaxel treatments in terms of tumor volume reduction of LLC1 xenografts growing subcutaneously.

**Methods:** Lewis Lung Carcinoma cells (LLC1) are a highly tumorigenic murine cell line initially derived from the lung of a C57BL mouse implanted with a primary Lewis Lung carcinoma. These cells can be subcutaneously grown in syngeneic C57BL6 mice, where they develop a very aggressive tumor and can be used to evaluate immunotherapy treatments. C57BL6 mice were injected with 0.25x10⁶ LLC1 cells in one flank to induce tumor formation. The day after tumor implantation, animals were distributed into treatments groups, and treatment started (n=5). ABTL0812 was administered by the oral route at 120 mg/kg/day; anti-PD1 antibody was administered intraperitoneally (i.p.) at 100 µg/dose every three days for up a total of five administrations; carboplatin and paclitaxel were administered i.p. at 15 and 5 mg/kg respectively, once a week, for a total of three four administrations. Tumor volume and body weight were monitored 3 times a week.

**Results:** ABTL0812 and anti-PD1+paclitaxel/carboplatin treatments significantly reduced tumor volume when compared to control animals (ANOVA followed by t-test *p<0.05). ABTL0812 efficacy was indeed similarly to the efficacy observed for docetaxel treatment. Interestingly, ABTL0812 potentiated the anti-tumor effect of docetaxel. Statistical analysis showed that this combination therapy significantly improves the reduction of tumor growth compared to docetaxel alone (p<0.001 by t-test). In addition, no decrease in body weight or hematological counts (not shown) were observed in any of the treatment groups, including those where ABTL0812 is administered with docetaxel, suggesting this combination had no toxic effects. See Figure 17 herein.

**Conclusion:** Literature on LLC1 xenografts describe that anti-PD1 treatment is not effective in these tumors, although the combination with paclitaxel/carboplatin shows significant tumor volume reduction compared with vehicle control group, due to the treatment with chemotherapy makes tumor cells immunogenic and recognizable by immune system, which is potentiated by anti-PD1 antibody. ABTL0812 administered alone shows similar efficacy to anti-PD1+paclitaxel/carboplatin, but when the triple combination ABTL0812+anti-PD1+paclitaxel/carboplatin is administered, it induces a significant tumor volume reduction compared with anti-PD1+paclitaxel/carboplatin, further demonstrating the potential synergism between ABTL0812, which also acts as a immunomodulator inducing a pro-inflammatory anti-tumoral tumor microenvironment which synergizes with immune checkpoint inhibitors to induce higher tumor volume reduction. These results suggest a combined therapy of ABTL0812 plus anti-PD1+paclitaxel/carboplatin, a standard treatment for lung cancer patients, could have a clinical interest for the treatment of lung cancer.

### 8.3: Anticancer activity of ABTL0812 alone or in combination with anti-PD1/paclitaxel/carboplatin in a murine model of lung cancer using LLC1 cells injected intraperitoneally in C57BL6 mice

**Objective:** Investigate the anti-tumor activity of ABTL0812 alone and in combination with anti-PD1 antibody and carboplatin/paclitaxel, a combinatory reference therapy for the treatment of human lung cancer, evaluating potential synergism between ABTL0812 and anti-PD1+carboplatin/paclitaxel treatments in terms of tumor volume reduction in LLC1 tumors growing intraperitoneally.

**Methods:** Lewis Lung Carcinoma cells (LLC1) are a highly tumorigenic murine cell line initially derived from the lung of a C57BL mouse implanted with a primary Lewis Lung carcinoma. These cells can be intraperitoneally grown in syngeneic C57BL6 mice, where they develop a very aggressive tumor attached to the intestine and can be used to evaluate immunotherapy treatments. C57BL6 mice were injected with 1x10⁶ LLC1 cells in the peritoneum to induce tumor formation. The day after tumor implantation, animals were distributed into treatments groups, and treatment started (n=2). ABTL0812 was administered by the oral route at 120 mg/kg/day; anti-PD1 antibody was administered i.p. at 100 ug/dose every three days for up a total of five administrations; paclitaxel and carboplatin were administered i.p. at 15 and 5 mg/kg respectively, once a week, for a total of three four administrations. Animals were euthanized after 14 days of treatment and tumors growing in the intestines were collected.

**Results:** ABTL0812 in combination with anti-PD1+paclitaxel/carboplatin significantly reduced tumor volume when compared to control, ABTL0812 and anti-PD1+paclitaxel/carboplatin treatments in a xenograft model of LLC1 cells growing intraperitoneally in C57BL6 mice, showing around half of the size. See Figure 18 herein.

**Conclusion:** LLC1 cells can be grown intraperitoneally in C57BL6 mice, developing a very aggressive tumor attached to the intestines. The triple combination ABTL0812+anti-PD1+paclitaxel/carboplatin reduces tumor growth compared to vehicle, ABTL0812 and anti-PD1+paclitaxel/carboplatin treatments. These results suggest a combined therapy of ABTL0812 plus anti-PD1+paclitaxel/carboplatin, a standard treatment for lung cancer patients, could have a clinical interest for the treatment of lung cancer.

### 8.4: Anticancer activity and tumor microenvironment immunomodulation mediated by ABTL0812 in a murine model of pancreatic cancer using MT5 cells implanted in C57BL6 mice

**Objective:** Investigate the anti-tumor activity and the tumor immunomodulatory *in vivo* effects of ABTL0812 alone and compared with anti-PD1 antibody, a reference drug for the treatment of different human cancer types. Anticancer efficacy will be assessed by tumor volume reduction and tumor microenvironment immunomodulation will be assessed by tumor immune cell infiltration analysis. Pembrolizumab is an anti-PD1 checkpoint inhibitor for human use - in this Example was used a corresponding modified version optimized for use in the murine model used in this Example.

**Methods:** MT5 cells are a highly tumorigenic murine cell line mutated in KRAS and p53 initially derived from the pancreas of triple transgenic KRAS-p53-Cre (KPC) mice, carrying pancreatic ductal adenocarcinoma. These cells can be subcutaneously grown in syngeneic C57BL6 mice, where they develop a very aggressive tumor and can be used to evaluate immunotherapy treatments. C57BL6 mice were injected with 2x10⁶ MT5 cells in one flank to induce tumor formation. When tumors had a volume of 50mm³ approximately, animals were homogenously randomized into treatment groups (n=9), and the different treatments were started. The treatment groups were Vehicle, ABTL0812 and anti-PD1. ABTL0812 was administered by the oral route at 480 mg/kg/day. Anti-PD1 antibody was administered i.p. at 200 µg/dose every three days. Tumor volume and body weight were monitored 3 times a week. At the end of the treatments, mice were euthanized, tumors collected and single cell suspension was obtained by digesting tumors using digestion media containing collagenase and lipase and further processing with trypsin and DNAse. Additionally, spleens from treated mice were collected, minced using a strainer, and single cells were obtained after treatment with trypsin and DNAse. After cell suspensions were obtained, cancer cells and immune cells infiltrated withing tumors were stained using specific antibodies against different immune cell subsets and further analyzed using flow cytometry. The combinations used were: Th1 cells = CD45+ CD4+ CCR4- CXCR3+, Th2 cells = CD45+ CD4+ CCR4+ CXCR3-, Myeloid cells = CD45+ CD11b+ Ly6C+ and NKs cells = CD45+ NK1.1+.

**Results:** ABTL0812 administered alone shows anticancer efficacy against MT5 tumors, significantly decreasing tumor volume compared with vehicle treatment group, and showing similar efficacy as anti-PD1 treatment administered alone. None of the treatments show any alteration in mice weight nor any clinical signs of toxicity, pain or suffering in the animals. Moreover, ABTL0812 induces an increase of Myeloid cells within tumors, correlating with its ability to potentiate M1 phenotypes *in vitro,* which is accompanied by an increase in the percentage of NK cells within tumor, cells with anticancer activity. Additionally, the spleen of ABTL0812 treated mice show and increase in the Th1/Th2 ratio, indicative of a pro-inflammatory immune system response *** p<0.001). See Figure 27 herein.

**Conclusion:** ABTL0812 shows anticancer efficacy in the murine pancreatic cancer model using MT5 cells through the modulation of tumor microenvironment towards a more pro-inflammatory and anti-tumoral environment. ABTL0812 increases the Th1/Th2 ratio in the spleen, indicative of a pro-inflammatory environment in the spleen, commonly used as an indicator or the mice immune system activation. As a consequence, ABTL0812 induces an increase of myeloid and NK cells within tumors, which indicates a pro-inflammatory anti-tumor immune infiltration. Importantly, this immunomodulatory effect mediated by ABTL0812 is significantly higher compared with anti-PD1 treatment. Pancreatic cancers are considered highly immunosuppressive and low immunogenic tumors, where immunotherapies administered alone do not show very optimistic effects. These data suggest that ABTL0812 is able to promote the transformation of cold pancreatic tumors into hot and more immunogenic tumors more efficiently than anti-PD1, therefore highlighting its potential combination with immunotherapies and chemotherapies to increase the anticancer efficacy.

### 8.5: Anticancer activity and tumor microenvironment immunomodulation mediated by ABTL0812 in combination with anti-PD1 and FOLFIRINOX in a murine model of pancreatic cancer using MT5 cells implanted in C57BL6 mice

**Objective:** Investigate the anti-tumor activity and the tumor immunomodulatory *in vivo* effects of ABTL0812 administered in combination with anti-PD1 and Folfirinox. Previous studies have shown the ability of ABTL0812 to potentiate the anticancer efficacy of Folfirinox, the standard treatment for human advanced pancreatic cancer patients, in a xenograft model of human pancreatic cancer using MiaPaca2 cells implanted in nude mice (Example 3.1). Based on the results of tumor microenvironment modulation mediated by ABTL0812 *in vivo* (Example 8.4), it was decided to test the triple combination to assess its efficiency in tumor volume reduction. Pembrolizumab is an anti-PD1 checkpoint inhibitor for human use - in this Example was used a corresponding modified version optimized for use in the murine model used in this Example.

**Methods:** As in Example 8.4, C57BL6 mice were injected with 2x10⁶ MT5 cells in one flank to induce tumor formation. When tumors had a volume of approximately 50 mm³, animals were homogenously randomized into treatment groups (n=9), and the different treatments were started. Treatment groups were vehicle, anti-PD1, ABTL0812 + Folfirinox, and the triple combination ABTL0812 + anti-PD1 + Folfirinox. ABTL0812 was administered by the oral route at 480 mg/kg/day. Anti-PD1 antibody was administered i.p. at 200 µg/dose every three days. Folfirinox chemotherapeutic combination was administered i.p. once a week for a total of four administrations. 5-FU at 30 mg/kg, leucovorin at 50 mg/kg, irinotecan at 50 mg/kg and oxaliplatin at 2.5 mg/kg were administered i.p. in two different days. 5-FU and leucovorin were administered on Tuesdays and irinotecan and oxaliplatin on Thursdays. Tumor volume and body weight were monitored 3 times a week. At the end of the treatments, mice were euthanized, tumors collected, and single cell suspension was obtained by digesting tumors using digestion media containing collagenase and lipase and further processing with trypsin and DNAse. After cell suspensions were obtained, cancer cells and immune cells infiltrated within tumors were stained using specific antibodies against different immune cell subsets and further analyzed using flow cytometry. The combinations used were: Myeloid cells = CD45+ CD11b+ Ly6C+ and CD8 cells = CD45+ CD3- CD8+.

**Results:** The triple combinatory treatment ABTL0812, anti-PD1 and FOLFIRINOX shows the highest anti-tumor effect with the most significant tumor volume reduction compared with the rest of the treatments. None of the treatments showed any alteration in mice weight nor any clinical signs of toxicity, pain or suffering in the animals. When tumor immune infiltration was analyzed, anticancer efficacy against MT5 tumors was associated to a significantly increase in myeloid and CD8 cells within tumors, showing anticancer activity and promoting a pro-inflammatory phenotype. None of the other treatments showed a significant increase in CD8 anticancer cells **** p<0.001). See Fig-ure 28 herein.

**Conclusion:** ABTL0812 in combination with anti-PD1 and FOLFIRINOX shows a potentiation of anticancer efficacy in the murine pancreatic cancer model using MT5 cells through the modulation of tumor microenvironment towards a more pro-inflammatory and anti-tumoral environment. ABTL0812 increases the myeloid cells within tumors, associated to an increase in CD8 anticancer immune cells, which could be translated into a more pro-inflammatory and anticancer environment. Pancreatic cancers are considered a highly immunosuppressive and low immunogenic tumor, were immunotherapies administered alone do not show very optimistic effects. These data suggest that the triple combination ABTL0812 + anti-PD1 and FOLFIRINOX could offer a more efficacious alternative to treat this type of cancer.

### REFERENCES

1: EP2409963B1 (Lipopharma - filed in 2010)
2: Erazo, et al.; Clinical Cancer Research; 22(10) May 15, 2016
3: WO2018/210830A1 (Ability Pharmaceuticals)

## Claims

1. A pharmaceutical combination comprising:
((A): a compound which is a polyunsaturated fatty acid of formula COO**R₁**-CH**R₂**-(CH₂)**a-**(CH=CHCH₂)**b**-(CH₂)**c**-CH₃, a pharmaceutically acceptable salt thereof, or a combination thereof, wherein
(i) **a** can be any integer value between 0 and 7,
(ii) **b** can be any integer value between 2 and 7,
(iii) **c** can be any integer value between 0 to 7,
(iv) **R₁** is H, Na, K, CH₃, CH₃-CH₂, or PO(O-CH₂-CH₃)₂, and
(v) **R₂** is OH, OCH₃, O-CH₂COOH, CH₃, Cl, CH₂OH, OPO(O-CH₂-CH₃)₂, N(OH)₂, F, HCOO or N(OCH₂CH₃)₂;
and
(B3): an immunotherapy agent compound
for the simultaneous, separate or sequential use in the treatment of a cancer in a human patient, wherein the treatment is an immunotherapy treatment of a cancer,
wherein (B3) is an immunotherapy agent compound which is a checkpoint inhibitor selected from the group consisting of:
- an anti-PD1 antibody, particularly wherein the anti-PD1 antibody is Nivolumab, Pembrolizumab or Spartalizumab; and
- an anti-PDL1 antibody, particularly wherein the anti-PDL1 antibody is Atezolizumab, Avelumab or Durvalumab; and
wherein Compound (A) is COOH-CHOH-(CH₂)₆-(CH=CH-CH₂)₂-(CH₂)₃-CH₃ (ABTL0812) or a pharmaceutically acceptable salt thereof.

2. The pharmaceutical combination for use according to claim 1, wherein
- the anti-PD1 antibody is Nivolumab, Pembrolizumab or Spartalizumab; and
- the anti-PDL1 antibody is Atezolizumab, Avelumab or Durvalumab.

3. The pharmaceutical combination for use according to claim 1, wherein Compound (B3) is an anti-PD1 antibody.

4. The pharmaceutical combination for use according to any of the preceding claims, wherein Compound (A) is a sodium salt of COOH-CHOH-(CH₂)₆-(CH=CH-CH₂)₂-(CH₂)₃-CH₃ (ABTL0812).

5. The pharmaceutical combination for use according to any of the preceding claims, wherein the cancer is at least one cancer selected from the group consisting of:
Lung cancer;
Non-small cell lung cancer;
Squamous cell cancer;
Adenocarcinoma cancer;
Endometrial cancer;
Endometrial serous cancer;
Endometroid cancer;
Pancreatic cancer;
Glioblastoma;
Resistant-recurrent breast cancer;
Head and neck cancer;
Multiple myeloma cancer;
Neuroblastoma;
Cholangiocarcinoma;
Colorectal cancer;
Larynx cancer;
Tongue cancer;
Prostate cancer;
Breast cancer;
Ovarian cancer;
Liver cancer;
Esophageal cancer;
Gall bladder cancer;
Bladder cancer; and
Gastric cancer.

6. The pharmaceutical combination for use according to any of the preceding claims, wherein the cancer is at least one cancer selected from the group consisting of:
Lung cancer;
Non-small cell lung cancer;
Squamous cell cancer;
Adenocarcinoma cancer;
Endometrial cancer;
Endometrial serous cancer;
Endometroid cancer;
Pancreatic cancer;
Glioblastoma;
Resistant-recurrent breast cancer;
Head and neck cancer;
Multiple myeloma cancer;
Neuroblastoma; and
Cholangiocarcinoma.

7. The pharmaceutical combination for use according to any of the preceding claims, wherein the pharmaceutical combination is a single composition comprising both Compound (A) and Compound (B3).

8. The pharmaceutical combination for use according to any of claims 1-6, wherein Compound (A) is administrated before administration of Compound (B3).

9. The pharmaceutical combination for use according to any of the preceding claims, wherein the pharmaceutical combination further comprises at least one Compound (B1), wherein Compound (B1) is a chemotherapeutic agent compound.

10. The pharmaceutical combination for use according to claim 9, wherein Compound (B1) is selected from the group consisting of:
Temozolomide;
Topotecan;
Irinotecan;
Cyclophosphamide;
Fluorouracil;
Cisplatin;
Carboplatin;
Oxaliplatin;
Leucovorin;
Doxorubicin;
Bleomycin;
Capecitabine;
Mitomycin B;
Paclitaxel;
Nab-paclitaxel;
Docetaxel;
Gemcitabine;
Methotrexate;
Pemetrexed;
Cytarabine;
Mercaptopurine;
Glufosfamide;
Ixabepilone;
Nimustine;
Carmustine;
Lomustine;
Mitoxantrone;
Etoposide;
Vincristine;
Vinblastine; and
Tamoxifen.

11. The pharmaceutical combination for use according to claim 9, wherein Compound (B1) is selected from the group consisting of:
Temozolomide;
Topotecan;
Irinotecan;
Cyclophosphamide;
Fluorouracil;
Oxaliplatin;
Leucovorin;
Doxorubicin;
Carboplatin; and
Paclitaxel.

12. The pharmaceutical combination for use according to claim 11, wherein Compound (B1) is paclitaxel and carboplatin.

13. The pharmaceutical combination for use according to claim 11, wherein Compound (B1) is irinotecan, leucovorin, oxaliplatin and fluorouracil.

14. The pharmaceutical combination for use according to claim 11, wherein Compound (B1) is paclitaxel and carboplatin, Compound (B3) is an anti-PD1 checkpoint inhibitor antibody, and the cancer is lung cancer or endometrial cancer.

15. The pharmaceutical combination for use according to claim 11, wherein Compound (B1) is irinotecan, leucovorin, oxaliplatin and fluorouracil, Compound (B3) is an anti-PD1 checkpoint inhibitor antibody, and the cancer is pancreatic cancer.

## Patentansprüche

1. Pharmazeutische Kombination, umfassend:
((A): eine Verbindung, die eine mehrfach ungesättigte Fettsäure der Formel COO**R₁**-CH**R₂**-(CH₂)**a**-(CH=CHCH₂)**b**-(CH₂)**c**-CH₃ ist, ein pharmazeutisch annehmbares Salz davon, oder eine Kombination davon, wobei
(i) **a** ein beliebiger ganzzahliger Wert zwischen 0 und 7 sein kann,
(ii) **b** ein beliebiger ganzzahliger Wert zwischen 2 und 7 sein kann,
(iii) **c** ein beliebiger ganzzahliger Wert zwischen 0 und 7 sein kann,
(iv) **R₁** ist H, Na, K, CH₃, CH₃-CH₂ oder PO(O-CH₂-CH₃)₂ und
(v) **R₂** ist OH, OCH₃, O-CH₂COOH, CH₃, Cl, CH₂OH, OPO(O-CH₂-CH₃)₂, N(OH)₂, F, HCOO oder N(OCH₂CH₃)₂; und
(B3): eine Immuntherapeutikum-Verbindung
zur gleichzeitigen, getrennten oder sequenziellen Verwendung bei der Behandlung eines Krebses in einem menschlichen Patienten, wobei die Behandlung eine immuntherapeutische Behandlung eines Krebses ist,
wobei (B3) eine Immuntherapeutikum-Verbindung ist, die ein Checkpoint-Inhibitor ist, ausgewählt aus der Gruppe bestehend aus:
- einem Anti-PD1-Antikörper, insbesondere wobei der Anti-PD1-Antikörper Nivolumab, Pembrolizumab oder Spartalizumab ist; und
- einem Anti-PDL1-Antikörper, insbesondere wobei der Anti-PDL1-Antikörper Atezolizumab, Avelumab oder Durvalumab ist; und
wobei Verbindung (A) COOH-CHOH-(CH₂)₆-(CH=CH-CH₂)₂-(CH₂)₃-CH₃ (ABTL0812) oder ein pharmazeutisch annehmbares Salz davon ist.

2. Pharmazeutische Kombination zur Verwendung nach Anspruch 1, wobei
- der Anti-PD1-Antikörper Nivolumab, Pembrolizumab oder Spartalizumab ist; und
- der Anti-PDL1-Antikörper Atezolizumab, Avelumab oder Durvalumab ist.

3. Pharmazeutische Kombination zur Verwendung nach Anspruch 1, wobei die Verbindung (B3) ein Anti-PD1-Antikörper ist.

4. Pharmazeutische Kombination zur Verwendung nach einem der vorhergehenden Ansprüche, wobei Verbindung (A) ein Natriumsalz von COOH-CHOH-(CH₂)₆-(CH=CH-CH₂)₂-(CH₂)₃-CH₃ (ABTL0812) ist.

5. Pharmazeutische Kombination zur Verwendung nach einem der vorhergehenden Ansprüche, wobei der Krebs mindestens ein Krebs ist, ausgewählt aus der Gruppe bestehend aus:
Lungenkrebs;
Nicht-kleinzelliges Lungenkarzinom;
Plattenzellkarzinom;
Adenokarzinom-Krebs;
Endometriumkarzinom;
Seröses Endometriumkarzinom;
Endometrioidkarzinom;
Bauchspeicheldrüsenkrebs;
Glioblastom;
Therapieresistenter rezidivierender Brustkrebs;
Kopf-Hals-Krebs;
Multiples-Myelom-Krebs;
Neuroblastom;
Cholangiokarzinom;
Kolorektaler Krebs;
Kehlkopfkrebs;
Zungenkrebs;
Prostatakrebs;
Brustkrebs;
Eierstockkrebs;
Leberkrebs;
Speiseröhrenkrebs;
Gallenblasenkrebs;
Blasenkrebs; und
Magenkrebs.

6. Pharmazeutische Kombination zur Verwendung nach einem der vorhergehenden Ansprüche, wobei der Krebs mindestens ein Krebs ist, ausgewählt aus der Gruppe bestehend aus:
Lungenkrebs;
Nicht-kleinzelliges Lungenkarzinom;
Plattenzellkarzinom;
Adenokarzinom-Krebs;
Endometriumkarzinom;
Seröses Endometriumkarzinom;
Endometrioidkarzinom;
Bauchspeicheldrüsenkrebs;
Glioblastom;
Therapieresistenter rezidivierender Brustkrebs;
Kopf-Hals-Krebs;
Multiples-Myelom-Krebs;
Neuroblastom; und
Cholangiokarzinom.

7. Pharmazeutische Kombination zur Verwendung nach einem der vorhergehenden Ansprüche, wobei die pharmazeutische Kombination eine einzige Zusammensetzung ist, die sowohl Verbindung (A) als auch Verbindung (B3) umfasst.

8. Pharmazeutische Kombination zur Verwendung nach einem der Ansprüche 1-6, wobei Verbindung (A) vor der Verabreichung von Verbindung (B3) verabreicht wird.

9. Pharmazeutische Kombination zur Verwendung nach einem der vorhergehenden Ansprüche, wobei die pharmazeutische Kombination ferner mindestens eine Verbindung (B1) umfasst, wobei die Verbindung (B1) eine Chemotherapeutikum-Verbindung ist.

10. Pharmazeutische Kombination zur Verwendung nach Anspruch 9, wobei Verbindung (B1) ausgewählt ist aus der Gruppe bestehend aus:
Temozolomid;
Topotecan;
Irinotecan;
Cyclophosphamid;
Fluorouracil;
Cisplatin;
Carboplatin;
Oxaliplatin;
Leucovorin;
Doxorubicin;
Bleomycin;
Capecitabin;
Mitomycin B;
Paclitaxel;
Nab-Paclitaxel;
Docetaxel;
Gemcitabin;
Methotrexat;
Pemetrexed;
Cytarabin;
Mercaptopurin;
Glufosfamid;
Ixabepilon;
Nimustin;
Carmustin;
Lomustin;
Mitoxantron;
Etoposid;
Vincristin;
Vinblastin; und
Tamoxifen.

11. Pharmazeutische Kombination zur Verwendung nach Anspruch 9, wobei Verbindung (B1) ausgewählt ist aus der Gruppe bestehend aus:
Temozolomid;
Topotecan;
Irinotecan;
Cyclophosphamid;
Fluorouracil;
Oxaliplatin;
Leucovorin;
Doxorubicin;
Carboplatin; und
Paclitaxel.

12. Pharmazeutische Kombination zur Verwendung nach Anspruch 11, wobei die Verbindung (B1) Paclitaxel und Carboplatin ist.

13. Pharmazeutische Kombination zur Verwendung nach Anspruch 11, wobei die Verbindung (B1) Irinotecan, Leucovorin, Oxaliplatin und Fluorouracil ist.

14. Pharmazeutische Kombination zur Verwendung nach Anspruch 11, wobei Verbindung (B1) Paclitaxel und Carboplatin ist, Verbindung (B3) ein Anti-PD1-Checkpoint-Inhibitor-Antikörper ist, und der Krebs Lungenkrebs oder Endometriumkarzinom ist.

15. Pharmazeutische Kombination zur Verwendung nach Anspruch 11, wobei Verbindung (B1) Irinotecan, Leucovorin, Oxaliplatin und Fluorouracil ist, Verbindung (B3) ein Anti-PD1-Checkpoint-Inhibitor-Antikörper ist und der Krebs Bauchspeicheldrüsenkrebs ist.

## Revendications

1. Combinaison pharmaceutique comprenant :
((A) : un composé qui est un acide gras polyinsaturé de formule COO**R₁-**CH**R₂**-(CH₂)**a**-(CH=CHCH₂)**b**-(CH₂)**c**-CH₃, un de ses sels pharmaceutiquement acceptable, ou une de leurs combinaisons, dans laquelle
(i) **a** peut être une quelconque valeur entière entre 0 et 7,
(ii) **b** peut être une quelconque valeur entière entre 2 et 7,
(iii) **c** peut être une quelconque valeur entière entre 0 et 7,
(iv) **R₁** est H, Na, K, CH₃, CH₃-CH₂, ou PO(O-CH₂-CH₃)₂, et
(v) **R₂** est OH, OCH₃, O-CH₂COOH, CH₃, CI, CH₂OH, OPO(O-CH₂-CH₃)₂, N(OH)₂, F, HCOO ou N(OCH₂CH₃)₂ ;
et
(B3) : un composé d'agent d'immunothérapie
pour l'utilisation simultanée, séparée ou séquentielle dans le traitement d'un cancer chez un patient humain, dans laquelle le traitement est un traitement d'immunothérapie d'un cancer,
dans laquelle (B3) est un composé d'agent d'immunothérapie qui est un inhibiteur de point de contrôle choisi dans le groupe constitué par :
- un anticorps anti-PD1, en particulier dans laquelle l'anticorps anti-PD1 est le Nivolumab, le Pembrolizumab ou le Spartalizumab ; et
- un anticorps anti-PDL1, en particulier dans laquelle l'anticorps anti-PDL1 est l'Atezolizumab, l'Avelumab ou le Durvalumab ; et
dans laquelle le Composé (A) est COOH-CHOH-(CH₂)₆-(CH=CH-CH₂)₂-(CH₂)₃-CH₃ (ABTL0812) ou un de ses sels pharmaceutiquement acceptables.

2. Combinaison pharmaceutique destinée à être utilisée selon la revendication 1, dans laquelle
- l'anticorps anti-PD1 est le Nivolumab, le Pembrolizumab ou le Spartalizumab ; et
- l'anticorps anti-PDL1 est l'Atezolizumab, l'Avelumab ou le Durvalumab.

3. Combinaison pharmaceutique destinée à être utilisée selon la revendication 1, dans laquelle le Composé (B3) est un anticorps anti-PD1.

4. Combinaison pharmaceutique destinée à être utilisée selon l'une quelconque des revendications précédentes, dans laquelle le Composé (A) est un sel de sodium de COOH-CHOH-(CH₂)₆-(CH=CH-CH₂)₂-(CH₂)₃-CH₃ (ABTL0812).

5. Combinaison pharmaceutique destinée à être utilisée selon l'une quelconque des revendications précédentes, dans laquelle le cancer est au moins un cancer choisi dans le groupe constitué par :
le cancer du poumon ;
le cancer du poumon non à petites cellules ;
le cancer épidermoïde ;
le cancer de type adénocarcinome ;
le cancer de l'endomètre ;
le cancer séreux de l'endomètre ;
le cancer endométrioïde ;
le cancer du pancréas ;
le glioblastome ;
le cancer du sein résistant-récidivant ;
le cancer de la tête et du cou ;
le cancer du myélome multiple ;
le neuroblastome ;
le cholangiocarcinome ;
le cancer colorectal ;
le cancer du larynx ;
le cancer de la langue ;
le cancer de la prostate ;
le cancer du sein ;
le cancer de l'ovaire ;
le cancer du foie ;
le cancer de l'oesophage ;
le cancer de la vésicule biliaire ;
le cancer de la vessie ; et
le cancer de l'estomac.

6. Combinaison pharmaceutique destinée à être utilisée selon l'une quelconque des revendications précédentes, dans laquelle le cancer est au moins un cancer choisi dans le groupe constitué par :
le cancer du poumon ;
le cancer du poumon non à petites cellules ;
le cancer épidermoïde ;
le cancer de type adénocarcinome ;
le cancer de l'endomètre ;
le cancer séreux de l'endomètre ;
le cancer endométrioïde ;
le cancer du pancréas ;
le glioblastome ;
le cancer du sein résistant-récidivant ;
le cancer de la tête et du cou ;
le cancer du myélome multiple ;
le neuroblastome ; et
le cholangiocarcinome.

7. Combinaison pharmaceutique destinée à être utilisée selon l'une quelconque des revendications précédentes, dans laquelle la combinaison pharmaceutique est une composition unique comprenant à la fois le Composé (A) et le Composé (B3).

8. Composition pharmaceutique destinée à être utilisée selon l'une quelconque des revendications 1 à 6, dans laquelle le Composé (A) est administré avant l'administration du Composé (B3).

9. Combinaison pharmaceutique destinée à être utilisée selon l'une quelconque des revendications précédentes, dans laquelle la combinaison pharmaceutique comprend en outre au moins un Composé (B1), dans laquelle le Composé (B1) est un composé agent chimiothérapeutique.

10. Combinaison pharmaceutique destinée à être utilisée selon la revendication 9, dans laquelle le Composé (B1) est choisi dans le groupe constitué par :
le témozolomide ;
le topotécan ;
l'irinotécan ;
le cyclophosphamide ;
le fluorouracile ;
le cisplatine ;
le carboplatine ;
l'oxaliplatine ;
la leucovorine ;
la doxorubicine ;
la bléomycine ;
la capécitabine ;
la mitomycine B ;
le paclitaxel ;
le nab-paclitaxel ;
le docétaxel ;
la gemcitabine ;
le méthotrexate ;
le pemetrexed ;
la cytarabine ;
la mercaptopurine ;
le glufosfamide ;
l'ixabepilone ;
la nimustine ;
la carmustine ;
la lomustine ;
la mitoxantrone ;
l'étoposide ;
la vincristine ;
la vinblastine ; et
le tamoxifène.

11. Combinaison pharmaceutique destinée à être utilisée selon la revendication 9, dans laquelle le Composé (B1) est choisi dans le groupe constitué par :
le témozolomide ;
le topotécan ;
l'irinotécan ;
le cyclophosphamide ;
le fluorouracile ;
l'oxaliplatine ;
la leucovorine ;
la doxorubicine ;
le carboplatine ; et
le paclitaxel.

12. Combinaison pharmaceutique destinée à être utilisée selon la revendication 11, dans laquelle le Composé (B1) est le paclitaxel et le carboplatine.

13. Combinaison pharmaceutique destinée à être utilisée selon la revendication 11, dans laquelle le Composé (B1) est l'irinotécan, la leucovorine, l'oxaliplatine et le fluorouracile.

14. Combinaison pharmaceutique destinée à être utilisée selon la revendication 11, dans laquelle le Composé (B1) est le paclitaxel et le carboplatine, le Composé (B3) est un anticorps inhibiteur du point de contrôle anti-PD1, et le cancer est un cancer du poumon ou un cancer de l'endomètre.

15. Combinaison pharmaceutique destinée à être utilisée selon la revendication 11, dans laquelle le Composé (B1) est l'irinotécan, la leucovorine, l'oxaliplatine et le fluorouracile, le composé (B3) est un anticorps inhibiteur du point de contrôle anti-PD1, et le cancer est un cancer du pancréas.
